# EUROPEAN PATENT APPLICATION

(11) **EP 1 860 443 A1**
(43) Date of publication of application: **28.11.2007**
(21) Application number: 06714960.9
(22) Date of filing: 01.03.2006
(51) Int. Cl.: G01N 35/08, G01N 27/414, G01N 37/00

(54) **MICROCHANNEL ARRAY AND METHOD FOR PRODUCING THE SAME, AND BLOOD MEASURING METHOD EMPLOYING IT**

(30) Priority: 07.03.2005 JP 2005062217
(71) Applicant: KURARAY CO., LTD., Kurashiki-shi, Okayama 710-8622 (JP)
(72) Inventor: NISHI, Taiji, Okayama 710-8622 (JP); FUKUDA, Motohiro, i, Ibaraki, 3050841 (JP); TAZAKI, Go, i, Ibaraki, 3050841 (JP); KANAI, Seiichi, i, Ibaraki, 3050841 (JP); KITANI, Takenori, i, Ibaraki, 3050841 (JP); FUKUHARA, Naoto, i, Ibaraki, 3050841 (JP)
(74) Representative: Vossius & Partner
(86) International application number: PCT/JP2006/303841
(87) International publication number: WO 2006/095615

(57) **Abstract**

A microchannel array with complex flow channels formed by a simple method, a method of manufacturing the same, and a blood test method are provided. The microchannel array is formed by joining the first substrate 10 and the second substrate 20, has the fluid inlet 1 and the fluid outlet 2 on its surface, and internally includes an internal space structure providing a connection from the fluid inlet 1 to the fluid outlet 2. The internal space structure includes the inlet-side space 3, the outlet-side space 4, the upstream flow channel 5 connected with the inlet-side space 3, the downstream flow channel 6 connected with the outlet-side space 4 and located opposite to the upstream flow channel 5 with a gap therebetween, and the micro flow channel 7 connecting between the upstream flow channel 5 and the downstream flow channel 6. The first substrate 10 and the second substrates 20 have depressions for creating the inlet-side space 3 and the outlet-side space 4 and grooves for creating the upstream flow channel 5 and the downstream flow channel 6, respectively, and the second substrates 20 has grooves for creating the micro flow channel 7.

## Description

### Technical Field

The present invention relates to a microchannel array, a method of manufacturing the same, and a blood test method using the same.

### Background Art

As societies mature, values on medical care and health have changed, and people now seek the "healthy and high-quality life", not merely the primary health care in a narrow range. It is expected that more and more individuals will place a higher value on preventive medicine than on curative medicine because of an increase in medical care costs, a fact that disease prevention is less costly than treatment, and an increase in the number of those who are in between healthy and diseased.

On this account, in the medical field, particularly in the clinical laboratory field, there is an increasing need for a non-restraint examination system that enables prompt examination and diagnosis in close proximity to a patient such as at an operating room, bedside and home, and for a noninvasive or minimally invasive examination system that requires only a small amount of sample of blood or the like.

The measurement and evaluation of formed components of blood, which are red blood cells, white blood cells and blood platelets, are essential for health care and diagnosis and treatment of diseases. In order to measure the red blood cell deformability, the ability of blood to pass through a film having minute openings such as a Nuclepore filter and a nickel mesh filter has been examined. For the measurement of platelet aggregability, a method of measuring a change in the turbidity of platelet suspension that accompanies the platelet aggregation has been used. Further, for the measurement of white blood cell activity, a Boyden chamber method, a particle phagocytosis test, a chemiluminescence method and so on have been used according to several aspects of the white blood cell activity. The white blood cell activity is particularly important for infection, immunotherapy, immunosuppressive therapy and so on.

However, the above measurement methods have problems such as low efficiency, low reproducibility and low quantitative ability, and therefore they fail to serve as effective measurement methods that are adequate for the importance of measurement. Further, the conventional platelet aggregability measurement method requires time and labor for sample preparation and its sensitivity is not sufficient.
Furthermore, the conventional red blood cell deformability measurement method is lack of reliability because openings or grooves can be obstructed by the formed components in a blood sample during measurement. This degrades the physiological or diagnostic values of the measurement results.

In order to eliminate the above problems, a technique of manufacturing a blood filter with the use of a semiconductor microfabrication technology has been proposed (Patent document 1). This technique performs patterning on a silicon substrate (first substrate 10) by photolithography, forms grooves on the silicon substrate by wet or dry etching, and then places a second substrate, which is a flat plate, on the surface having the grooves, thereby creating blood flow channels. This technique enables designing of a ratio of a flow channel width and a flow channel depth of minute flow channels, an interval or the like depending on the purpose.

[Patent document 1] Japanese Patent No. 2685544

### Disclosure of the Invention

### Problems to be Solved by the Invention

Blood is classified broadly into blood cell (formed) components and blood plasma (fluid) components. The percentage of the blood cell components is about 40% to 45% and that of the blood plasma components is about 55% to 60%. The blood cell components are composed of about 96% of red blood cells and about 4% of white blood cells and blood platelets. A red blood cell has a diameter of about 7 to 8 µm, a white blood cell has a diameter of about 12 to 14 µm, and a blood platelet has a diameter of about 3 µm. With the use of the semiconductor microfabrication technology that is described in the above-mentioned Patent document 1, it is possible to form a micro flow channel by creating microgrooves having various shapes and sizes adequate for the shapes of red blood cells, white blood cell and blood platelets on a silicon substrate and then placing a flat plate on top of the substrate. Practically, however, it is necessary to form a deeper flow channel or space at the front and back of a micro flow channel. This is because it is extremely difficult to let the blood flow through the micro flow channel with a width or depth of about 3 to 14 µm due to the resistance caused by the surface viscosity. A minute amount of blood sample fails to reproduce the state in a living body when dried, and a micro flow channel can be occluded by a thrombus.

The formation of a deeper flow channel or space at the front and back of a micro flow channel requires formation of a larger flow channel or depression at the front and back of the micro flow channel. It is thus necessary to create a mult-level structure in the depth direction. This needs to perform photolithography and etching using alignment two or more times.

Further, the reproduction of a flow in a biological model requires formation of grooves having a wide flow channel as a main blood vessel, a branch flow channel as a tributary, and a minute flow channel as a capillary on one substrate. This needs to further repeat the photolithography and etching using alignment. However, it is extremely difficult to realize a flow that reproduces a biological model because of limits on manufacture as well as costs.

Although the problems when a microchannel array is applied to a blood test are described above, it is not limited thereto, and similar problems can occur when it is applied to other tests.

The present invention has been accomplished to solve the above problems and an object of the present invention is thus to provide a microchannel array with more complex flow channels formed by a simple method, a method of manufacturing the same, and a blood test method using the same.

### Means for solving the problems

The inventors of the present invention have conducted intensive studies and found that the object of the present invention can be achieved by the following aspects.
A microchannel array according to the present invention is a microchannel array formed by adhering or joining a first substrate and a second substrate, having a fluid inlet and a fluid outlet on a surface, and internally having an internal space structure providing a connection from the fluid inlet to the fluid outlet, wherein the internal space structure includes at least one upstream flow channel connected with the fluid inlet; at least one downstream flow channel connected with the fluid outlet and located opposite to the upstream flow channel with a gap therebetween; and a micro flow channel connecting between the upstream flow channel and the downstream flow channel, a minimum distance from a center of a sectional surface of the flow channel to a side wall of the flow channel being smaller than that of the upstream flow channel and the downstream flow channel, each of surfaces of the first substrate and the second substrate to be adhered or joined together has grooves for creating the upstream flow channel and the downstream flow channel, and the surface of the second substrate to be adhered or joined with the first substrate has a groove for creating the micro flow channel.

The microchannel array according to a first aspect of the present invention provides microfabrication on both of the first substrate and the second substrate, thus enabling creation of more complex flow channels.

A method of manufacturing a microchannel array according to the present invention includes forming a first substrate and a second substrate by a step of forming a resist pattern on a substrate, a step of forming a metal structure by depositing a metal over the resist pattern formed on the substrate, and a step of forming a molded article using the metal structure as a mold; and adhering or joining the first substrate and the second substrate.

The manufacturing method of the microchannel array according to the present invention provides microfabrication on both of the first substrate and the second substrate and adheres or joins the substrates together, thus enabling creation of more complex flow channels compared with the case of providing microfabrication on one substrate only. This also enables elimination or reduction of alignment process, thereby achieving cost reduction.

A blood test method according to a first aspect of the present invention uses the microchannel array of the above aspect and includes bringing a sample at least containing a blood sample to flow into a micro flow channel formed in an internal space structure in the microchannel array from a fluid inlet of the microchannel array; measuring a state of each blood component of the blood passing through the micro flow channel; and obtaining flow characteristics or activity of each blood component of the blood by the measurement.

A blood test method according to a second aspect of the present invention uses the microchannel array of the above aspect and includes making a difference in concentration of a biologically active substance between an inlet and an outlet of a micro flow channel formed in the microchannel array to enhance movement of a white blood cell through the micro flow channel; and measuring fluctuations in the number of white blood cell fractions at the inlet or the outlet of the micro flow channel or in the micro flow channel, or an occluded state of the micro flow channel due to a white blood cell; and obtaining migrability and adhesibility of a white blood cell fraction by the measurement.

A blood test method according to a third aspect of the present invention uses the microchannel array of the above aspect and includes coloring either one of a blood cell and a fluid component of the blood with a luminescent or fluorescent substance; bringing a sample at least containing a blood sample to flow into a micro flow channel formed in an internal space structure in the microchannel array from a fluid inlet of the microchannel array; measuring light intensity of each blood component of the blood passing through the micro flow channel; and obtaining activity of the measured blood component from a value of the light intensity.

A blood test method according to a fourth aspect of the present invention uses the microchannel array of the above aspect and includes depositing a thin film such as gold on at least a part of a wall surface of an internal space structure of the microchannel array, and bringing a sample at least containing a blood sample to flow into a micro flow channel formed in the internal space structure in the microchannel array from a fluid inlet of the microchannel array; and measuring a change in dielectric constant before and after passing through the micro flow channel as a change in intensity of reflected light due to surface plasmon resonance, and obtaining activity of a blood cell component from a measurement value.

A blood test method according to a fifth aspect of the present invention uses the microchannel array of the above aspect and includes placing a sensor for detecting a small frequency change by ultrasound on one of wall surfaces of an internal space structure of the microchannel array, and bringing a sample at least containing a blood sample to flow into a micro flow channel formed in the internal space structure in the microchannel array from a fluid inlet of the microchannel array; and measuring a frequency change before and after passing through the micro flow channel, and obtaining activity of a blood cell component from a measurement value.

A blood test method according to a sixth aspect of the present invention uses the microchannel array of the above aspect and includes placing a FET sensor on one of wall surfaces of an internal space structure of the microchannel array, and bringing a sample at least containing a blood sample to flow into a micro flow channel formed in the internal space structure in the microchannel array from a fluid inlet of the microchannel array; and measuring a small electrical displacement before and after passing through the micro flow channel, and obtaining activity of a blood cell component from a measurement value.

A blood test method according to a seventh aspect of the present invention uses the microchannel array of the above aspect and includes placing an electrode on one of wall surfaces of an internal space structure of the microchannel array and fixing a reagent; and bringing a sample at least containing a blood sample to flow into a micro flow channel from a fluid inlet of the microchannel array to mix the blood sample with the reagent, measuring a small electrical displacement after a chemical change, and obtaining biochemical data.

A blood test method according to an eighth aspect of the present invention uses the microchannel array of the above aspect and includes fixing a reagent onto at least a part of a wall surface of an internal space structure of the microchannel array; bringing a sample at least containing a blood sample to flow into a micro flow channel from a fluid inlet of the microchannel array to mix the blood sample with the reagent, and then applying light to the microchannel array; and measuring a variation before and after light application and obtaining biochemical data.

The blood test method according to the present invention can estimate the flow of a microcirculation system in a living body by obtaining the flow characteristics or activity of blood components flowing through the micro flow channel. This enables the prediction of the development of lifestyle-related diseases and the provision of guidance for healthy lifestyle habits based on the estimated flow and occluded state.

### Advantages of the Invention

The present invention can provide a microchannel array having more complex flow channels that are formed by a simple method, a method of manufacturing the same, and a blood test method using the same.

### Brief Description of the Drawings

[Fig. 1A] A perspective view of a microchannel array according to an embodiment.
[Fig. 1B] A view showing elements of Fig. 1A on a larger scale.
[Fig. 2A] A top view of a first substrate of a microchannel array according to an embodiment (example).
[Fig. 2B] A cross-sectional view along line IIB-IIB' in Fig. 2A.
[Fig. 3A] A top view of a second substrate of a microchannel array according to an embodiment (example).
[Fig. 3B] A cross-sectional view along line IIIB-IIIB' in Fig. 3A.
[Fig. 4A] An explanatory view showing a manufacturing process of a microchannel array according to an embodiment.
[Fig. 4B] An explanatory view showing a manufacturing process of a microchannel array according to an embodiment.
[Fig. 4C] An explanatory view showing a manufacturing process of a microchannel array according to an embodiment.
[Fig. 4D] An explanatory view showing a manufacturing process of a microchannel array according to an embodiment.
[Fig. 4E] An explanatory view showing a manufacturing process of a microchannel array according to an embodiment.
[Fig. 4F] An explanatory view showing a manufacturing process of a microchannel array according to an embodiment.
[Fig. 4G] An explanatory view showing a manufacturing process of a microchannel array according to an embodiment.
[Fig. 4H] An explanatory view showing a manufacturing process of a microchannel array according to an embodiment.
[Fig. 5A] A top view of a first substrate of a microchannel array B according to an example.
[Fig. 5B] A cross-sectional view along line VB-VB' in Fig. 5A.
[Fig. 6] A top view of a first substrate of a microchannel array C according to an example.
[Fig. 7] A top view of a second substrate of a microchannel array C according to an example.
[Fig. 8A] A top view of a second substrate of a microchannel array D according to an example.
[Fig. 8B] A cross-sectional view along line VIIIB-VIIIB' in Fig. 8A.
[Fig. 9] An image showing a flow passing through a micro flow channel in a blood test.
[Fig. 10A] A top view of a first substrate of a microchannel array X according to a comparative example.
[Fig. 10B] A cross-sectional view along line XB-XB' in Fig. 10A.

### Description of Reference Numerals

- 1: Fluid inlet
- 2: Fluid outlet
- 3: Inlet-side space
- 4: Outlet-side space
- 5: Upstream flow channel
- 6: Downstream flow channel
- 7: Micro flow channel
- 10: First substrate
- 11: Bank portion
- 13: Inlet-side first space
- 14: Outlet-side first space
- 15: Upstream first grooves
- 16: Downstream first grooves
- 18: First alignment portion
- 19: Second alignment portion
- 20: Second substrate
- 23: Inlet-side second depression
- 24: Outlet-side second depression
- 25: Upstream second grooves
- 26: Downstream second grooves
- 27: Microgrooves
- 28: Third alignment portion
- 29: Fourth alignment poriton
- 31: Substrate
- 32: First resist layer
- 33: Mask A
- 34: Second resist layer
- 35: Mask B
- 36: Resist pattern
- 37: Conductive film
- 38: Metal structure
- 39: Resin plate
- 100: Microchannel array

### Best Modes for Carrying Out the Invention

An example of an embodiment of the present invention is described hereinafter. Other embodiment are also included within the scope of the present invention as long as they do not deviate from the gist of the present invention.

### [Microchannel array]

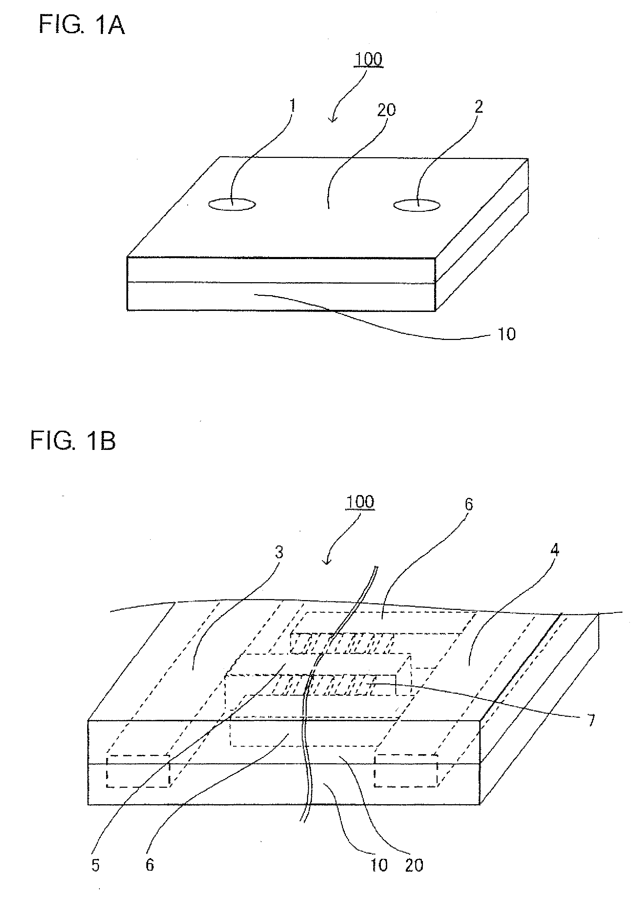

Fig. 1A is a perspective view of a microchannel array according to this embodiment, and Fig. 1B is a perspective view showing elements of the microchannel array of this embodiment on a larger scale. As shown in Fig. 1A, a microchannel array 100 of this embodiment includes a first substrate 10, a second substrate 20, a fluid inlet 1, and a fluid outlet 2. As shown in Fig. 1B, it internally includes an inlet-side space 3, an outlet-side space 4, an upstream flow channel 5, a downstream flow channel 6, and a micro flow channel 7, which constitutes an internal space structure. The internal space structure provides a connection from the fluid inlet 1 to the fluid outlet 2. In Fig. 1B, illustration of alignment portions, which are described later, are omitted for convenience of description.

In the microchannel array 100, the principal surfaces of the first substrate 10 and the second substrate 20 are adhered or joined to each other so as to form an integral structure. Materials of the first substrate 10 and the second substrate 20 are not particularly limited. For example, a glass substrate, a silicon substrate, or a resin substrate may be used. The use of a resin substrate is preferred. The reason is described later. In this embodiment, the first substrate 10 and the second substrate 20 are both a resin substrate with a vertical dimension of 15 mm, a horizontal dimension of 15 mm, and a thickness of 1 mm.

The fluid inlet 1 and the fluid outlet 2 are formed on the surface of the second substrate 20. In this embodiment, the fluid inlet 1 is located in close proximity to one side of the second substrate 20, and the fluid outlet 2 is located in close proximity to one side opposite to the side close to the fluid inlet 1. The fluid inlet 1 is an entrance portion into which a blood sample or the like is injected. In this embodiment, the outer diameter of the fluid inlet 1 and the fluid outlet 2 is 1.6 mm.

The inlet-side space 3 is in connection with the fluid inlet 1, and the outlet-side space 4 is in connection with the fluid outlet 2. A plurality of upstream flow channels 5 and a plurality of downstream flow channels 6 are formed in parallel with each other in an alternating sequence. Further, a plurality of micro flow channels 7 that establish a connection between the adjacent upstream flow channel 5 and the downstream flow channel 6 are formed substantially orthogonal to the upstream flow channel 5 and the downstream flow channel 6. The upstream flow channel 5 is in connection with one side surface of the inlet-side space 3, and the downstream flow channel 6 is in connection with one side surface of the outlet-side space 4. In such a structure, a blood sample or the like comes from the fluid inlet 1 as headstream, flows through the inlet-side space 3, the upstream flow channel 5, the micro flow channel 7, the downstream flow channel 6 and the outlet-side space 4, to reach the fluid outlet 2. The micro flow channel 7 serves as a place to observe the fluency of a blood sample, the occluded state and so on. This is described in detail later.

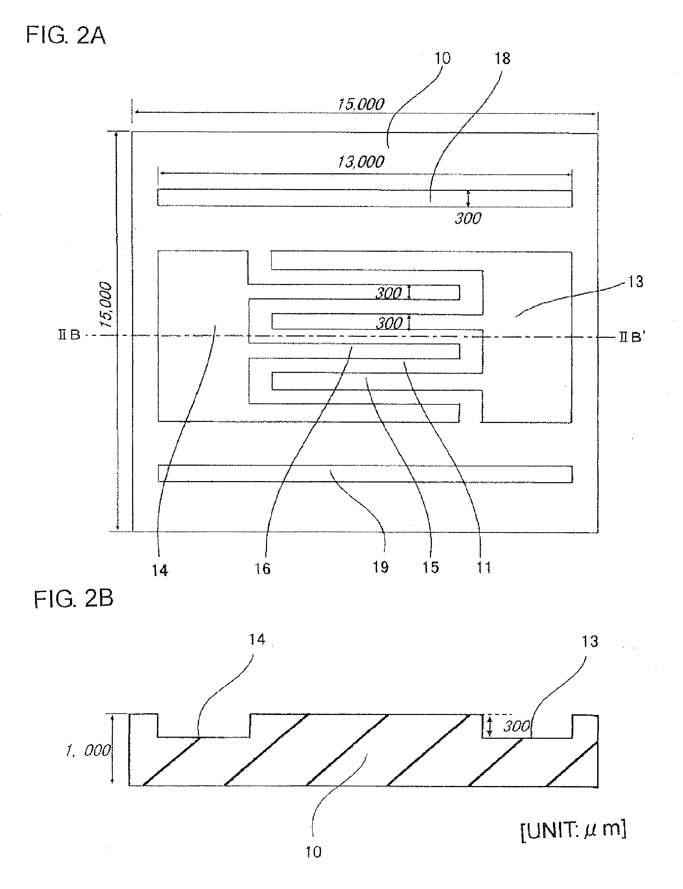

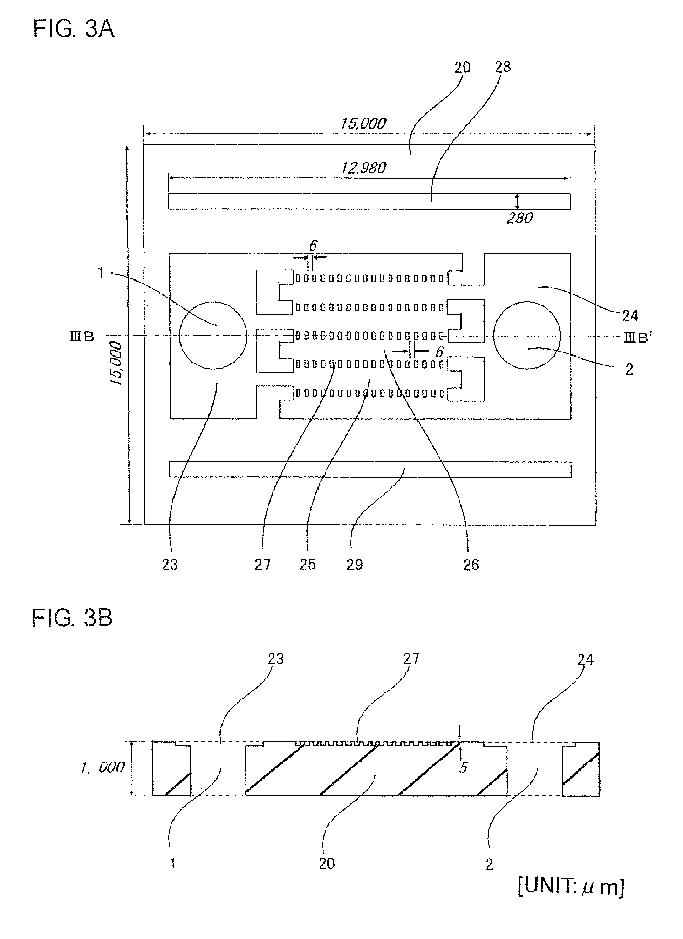

The structure of the first substrate 10 and the second substrate 20 is described in detail hereinbelow. Fig. 2A is a top view of the surface of the first substrate 10 of this embodiment to be in contact with the second substrate 20, and Fig. 2B is a cross-sectional view along line IIB-IIB' in Fig. 2A. Fig. 3A is a top view of the surface of the second substrate 20 of this embodiment to be in contact with the first substrate 10, and Fig. 3B is a cross-sectional view along line IIIB-IIIB' in Fig. 3A.

On the surface of the first substrate 10 which faces the second substrate 20, grooves and depression regions as shown in Fig. 2A are formed. Specifically, it has an inlet-side first depression 13, an outlet-side first depression 14, upstream first grooves 15, downstream first grooves 16, a first alignment portion 18, and a second alignment portion 19. The depths of the depressions and grooves are the same (300 µm in this embodiment). This enables reduction of manufacturing process and costs. The "depth" is a length along the thickness of a substrate.

The inlet-side first depression 13 is located in close proximity to one side of the first substrate 10, and the outlet-side first depression 14 is located in close proximity to one side opposite to the side close to the inlet-side first depression 13. The inlet-side first depression 13 is an area through which a blood sample or the like that is injected from the fluid inlet 1 passes firstly when joined or adhered to the second substrate 20, and it is a part of the inlet-side space 3. The outlet-side first depression 14 is an area through which a blood sample or the like having passed through a flow channel or the like passes immediately before it reaches an outlet as an exit portion when joined or adhered to the second substrate 20, and it is a part of the outlet-side space 4.

There are three upstream first grooves 15 and three downstream first grooves 16, and they are arranged in an alternating sequence and in parallel with each other. A flow channel width of each of the upstream first grooves 15 and the downstream first grooves 16 is 300 µm. In the following description, a gap between the upstream first grooves 15 and the downstream first grooves 16 is referred to as a bank portion 11. The three upstream first grooves 15 are connected with the inlet-side first depression 13 on one side of the inlet-side first depression 13 which faces the outlet-side first depression 14. Likewise, the three downstream first grooves 16 are connected with the outlet-side first depression 14 on one side of the outlet-side first depression 14 which faces the inlet-side first depression 13. The upstream first grooves 15 is a part of the upstream flow channel 5 which is to be formed when joined or adhered to the second substrate 20. The downstream first grooves 16 is a part of the downstream flow channel 6 which is to be formed when joined or adhered to the second substrate 20.

The flow channel width and the flow channel depth of the upstream flow channel 5 and the downstream flow channel 6 are preferably in the range of 20 to 1000 µm, more preferably in the range of 30 to 500 µm, for the purpose of letting a sample to be measured flow smoothly and preventing coagulation, deactivation or the like of a sample due to drying. The ratio of the flow channel width and the flow channel depth of the upstream flow channel 5 and the downstream flow channel 6 is preferably selected from the range of 1:10 to 10:1 depending on the viscosity of a sample to be measured.

The first alignment portion 18 and the second alignment portion 19 are regions for the alignment with the second substrate 20. In this embodiment, they have the shape of depressions which are located outside of the upstream first grooves 15 and the downstream first grooves 16 in parallel therewith.

The second substrate 20 is described hereinbelow. As shown in Figs. 3A and 3B, the second substrate 20 has grooves and depression regions. Specifically, it has the fluid inlet 1, the fluid outlet 2, an inlet-side second depression 23, an outlet-side second depression 24, upstream second grooves 25, downstream second grooves 26, microgrooves 27, a third alignment portion 28, and a fourth alignment portion 29. The depression or groove depths of the inlet-side second depression 23, the outlet-side second depression 24, the upstream second grooves 25, the downstream second grooves 26, and the microgrooves 27 are all 5 µm. This enables reduction of manufacturing process and costs. The third alignment portion 3 and the fourth alignment portion have projected patterns to fit with the first alignment portion and the second alignment portion.

The inlet-side second depression 23 is configured to overlap the inlet-side first depression 13 when the first substrate 10 and the second substrate 20 are jointed or adhered, being opposed to each other. The outlet-side second depression 24 is configured to overlap the outlet-side first depression 14 when the first substrate 10 and the second substrate 20 are jointed or adhered, being opposed to each other. Thus, the inlet-side second depression 23 is located in close proximity to one side of the second substrate 20, and the outlet-side second depression 24 is located in close proximity to one side opposite to the side close to the inlet-side second depression 23. When the first substrate 10 and the second substrate 20 are jointed or adhered together, the inlet-side first depression 13 and the inlet-side second depression 23 form the inlet-side space 3. Further, the outlet-side first depression 13 and the outlet-side second depression 24 form the outlet-side space 4.

As shown in Fig. 3B, the fluid inlet 1 and the fluid outlet 2 have through holes that provides a connection from the surface of the second substrate 20 on the opposite side of the surface facing the first substrate 10 to the base of the inlet-side second depression 23 and the base of the outlet-side second depression 24, respectively. In this embodiment, the diameter of the through holes is 1.6 mm.

The upstream second grooves 25 is located in the position that faces the upstream first grooves 15 when the first substrate 10 and the second substrate 20 are jointed or adhered, being opposed to each other. Specifically, three parallel flow channels are connected with the inlet-side second depression 23 on one side of the inlet-side second depression 23 which faces the outlet-side second depression 24. On the other hand, the downstream second grooves 26 is located in the position that faces the downstream first grooves 16 when the first substrate 10 and the second substrate 20 are jointed or adhered, being opposed to each other. Specifically, three parallel flow channels are connected with the outlet-side second depression 24 on one side of the outlet-side second depression 24 which faces the inlet-side second depression 23.

The microgrooves 27 are arranged in each gap between the upstream second grooves 25 and the downstream second grooves 26 so as to establish a connection therebetween. The width of the microgrooves is 6 µm in this embodiment. When the first substrate 10 and the second substrate 20 are jointed or adhered, the microgrooves 27 are opposite to the bank portion 11 of the first substrate 10, thereby forms the micro flow channel 7. The micro flow channel 7 is located substantially orthogonal to the upstream second grooves 25 and the downstream second grooves 26. This allows a blood sample which inflows from the upstream flow channel to flow into a larger number of micro flow channels, so that an observer can keep track of the flow of the blood sample, the occluded state and so on based on the overall condition of the micro flow channel. It is thereby possible to perform a more accurate blood test. The direction of the micro flow channel 7 is not limited to substantially orthogonal to the upstream flow channel 5, and it may be tilted from the orthogonal direction depending on the purpose of a use or application. The length of a wall that divides the adjacent microgrooves 27 can vary according to need. The flow channel length of the micro flow channel 7 can be thus adjusted appropriately.

A blood sample can be agglutinated by contact with a material or air when it is collected from a test subject even with the addition of an anticoagulant agent such as heparin. If the number of the microgrooves 27 is small, that is, if the number of the micro flow channels 7 is small, there is a risk of erroneous diagnosis at the sight of a certain minute microaggregate that is occluded by a blood aggregate before a blood test. Therefore, the number of the microgrooves 27 is preferably large for more accurate diagnosis.

The third alignment portion 28 and the fourth alignment portion 29 are formed in projected patterns and located in the positions that face the first alignment portion 18 and the second alignment portion 19, respectively, when the first substrate 10 and the second substrate 20 are placed opposite to each other. Specifically, they are located outside of the upstream second grooves 25 and the downstream second grooves 26 in parallel with the upstream second grooves 25 and the downstream second grooves 26, respectively. The height of the projected pattern is 250 µm. Alignment is performed with the use of these projected patterns and the depressed patterns of the first alignment portion 18 and the second alignment portion 19.

The first substrate 10 and the second substrate 20 are adhered or jointed be performing the alignment such that the first alignment portion 18 overlaps the third alignment portion 28 and the second alignment portion 19 overlaps the fourth alignment portion 29. The inlet-side first depression 13 and the inlet-side second depression 23 thereby overlap to form the inlet-side space 3. Likewise, the outlet-side first depression 14 and the outlet-side second depression 24 overlap to form the outlet-side space 4. Further, the upstream first grooves 15 and the upstream second grooves 25 overlap to integrally form the upstream flow channel 5, and the downstream first grooves 16 and the downstream second grooves 26 overlap to integrally form the downstream flow channel 6. The bank portion 11 which is formed on the first substrate 10 and divides the upstream first grooves 15 and the downstream first grooves 16 is placed opposite to the microgrooves 27 which is formed on the second substrate 20, thereby forming the micro flow channel 7.

The flow channel width and the flow channel depth of the micro flow channel 7 are preferably selected from the range of 1 to 50 µm and more preferably within the range of 1 to 20 µm depending on a sample to be measured, e.g. a blood cell component of a blood sample. The ratio of the flow channel width and the flow channel depth of the micro flow channel 7 is preferably selected from the range of 1:10 to 10:1 depending on the shape and deformability of a target blood cell component.

In such a structure, the microchannel array 100 has the internal space structure in which the inlet-side space 3, the upstream flow channel 5, the micro flow channel 7 and to the downstream flow channel 6 are connected.

Although a material of the first substrate and the second substrate that are used for the microchannel array 100 is not particularly limited as described above, a resin material is preferred in terms of a material cost and surface treatment efficiency. In the case of observing a blood cell by chemiluminescence or fluorometry, for example, a highly transparent resin material is preferred for the observation with the use of a fluorescence microscope, for example. Although a resin material is not particularly limited, acrylic resin, polylactide resin, polyglycolic acid resin, styrene resin, acrylic-styrene copolymer (MS resin), polycarbonate resin, polyester resin such as polyethylene terephthalate, polyvinyl alcohol resin, ethylene-vinyl alcohol copolymer, thermoplastic elastomer such as styrene elastomer, vinyl chloride resin, or silicone resin such as polydimethylsiloxane, vinyl acetate resin (product name: "Exceval"), polyvinyl butyral resin and so on may be used, for example.

Such a resin may contain one or more than one agent of lubricant, light stabilizer, heat stabilizer, antifogging agent, pigment, flame retardant, antistatic agent, mold release agent, antiblocking agent, ultraviolet absorbent, antioxidant and so on according to need.

In the case of using a microchannel array for a blood test and employing an optical detection method, a transparent substrate is used. For example, in the observation of actual conditions using a CCD camera or the like, either one or both of the first substrate 10 and the second substrate 20 is transparent. In the observation of reflected light, a substrate on the side of an optical system is a transparent plate, and a substrate on the opposite side is an opaque plate. An opaque substrate may be prepared by selecting an opaque grade at the stage of material selection or by depositing an inorganic film such as aluminum using deposition, for example, on the front surface or back surface of a transparent substrate. The optical properties for defining transparency are preferably a light transmittance of 80% or higher and a haze value of 10% or lower in a plate with a thickness of 1 mm. Further, when using an optical detection method, it is preferred to select an appropriate material depending on the wavelength of light used, such as using a material that does not contain ultraviolet absorbent or using a material that does not have a ring structure in a molecule.

A microchannel array preferably has a small difference in wettability from a water-type fluid such as physiological saline, blood sample or reagent to be in contact. If a difference in wettability is large, it is highly possible that a water-type fluid does not flow through a flow channel. Further, air bubbles can enter when filling a flow channel with physiological saline, for example, before performing a blood test, thus failing to maintain the same measurement value of a passage time of a target blood cell component. Furthermore, because cells are normally subject to immobilization onto a hydrophobic surface, it is likely in blood cells that blood cell components are attached to a flow channel, which causes problems such as failing to flow.

When performing a blood test with the use of a microchannel array, in order to suppress activation of a blood platelet, which is a factor of blood coagulation, and suppress the adhesion onto a material surface, the use of a material for sustained release of heparin, which is a medical agent for preventing blood platelet adhesion, a material of immobilized urokinase, which is enzyme, a material with a hydrophilic surface, and a material having a microphase-separated structure, is known. The use of a material with a hydrophilic surface is particularly preferred as a material that satisfies costs and performance constraints.

A generally-used thermoplastic resin such as polymethyl methacrylate normally has a relatively large contact angle with respect to water (for example, about 68° for polymethyl methacrylate resin, about 70° for polycarbonate resin, and 84° for polystyrene resin). It is thus necessary to reduce a contact angle with water. A technique for modifying the wettability of such a plastic surface is described hereinafter. In a blood test, a contact angle of a microchannel array surface with respect to water is preferably 0.5° to 60°, and more preferably 1° to 50°. If it is outside this range, it is difficult to bring a blood sample into a microgroove, and it fails to obtain stable data in the measurement of a passage time of blood cells or the like because of the presence of aggregates due to the adhesion of blood cells. It is therefore preferred that a contact angle is within the above range.

The use of a resin microchannel array has an advantage of allowing incineration as infectious waste, just like a thermoplastic resin such as a circuit that is used for blood purification treatment including artificial dialysis and plasma exchange. On the other hand, the use of a silicon plate that is formed by etching is made of an inorganic material and not incinerable. Landfill disposal as industrial waste requires sterilization and results in high costs. This is also against the increasing awareness of environmental issues.

A microchannel array can cope with an increase in the amount of waste accompanying a future increase in disposable products because of its incinerability, and the use of a resin material for a substrate to overlap eliminates the need for separation and permits the incineration all together. Furthermore, the use of a thermoplastic resin that does not contain halogen, such as polymethyl methacrylate, prevents the generation of harmful dioxin to allow easy incineration in an incinerator at a temperature normally used for the incineration of non-industrial waste and enables reuse as a heat resource.

In a microchannel array of this embodiment, the internal space structure is formed by providing microfabrication on both of the first substrate 10 and the second substrate 20 and then adhering or joining the micro-fabricated surfaces of the substrates together, thereby providing a fine space structure with a simple method.

In the case of using a microchannel array for a blood test, in order to bring blood into a micro flow channel and actually let the blood flow therethrough, it is necessary to form deeper flow channels at the front and back of the micro flow channel. In the case of forming a microgroove and deeper upstream flow channel and downstream flow channel at the front and back of the microgroove on either one of the two substrates as described in the above-mentioned Patent document 1, it is necessary to perform alignment for ensuring the positional accuracy and then carry out etching processes two times. This complicates a manufacturing process and increases processing costs. In this embodiment, the first substrate 10 and the second substrate 20 are provided with depressions and grooves, each having a uniform processing depth. It thus requires only one photolithography process for each substrate. Further, there is no need to perform alignment for ensuring the positional accuracy between the microgrooves and the upstream flow channel, the down stream flow channel or the like. It is thus advantageous in processing costs.

Although the depth of the depressions and grooves in the first substrate 10 and the second substrate 20 are uniform in the microchannel array 100 described above, it is not limited thereto, and the depth of the depressions and grooves in the first substrate 10 and/or the second substrate 20 may be respectively a multilevel structure. Further, a multilevel structure may be provided at the front and back of the micro flow channel 7 of the second substrate 20.

Depending on the flow channel width or the flow channel length of the micro flow channel, when letting a blood sample flow from the upstream flow channel 5, which is a main blood vessel as a headstream, to the micro flow channel 7, which is a capillary blood vessel, the blood sample flows into an extremely narrow flow channel, so that the activation of blood platelets occurs to cause the occlusion of the micro flow channel even if it is a blood sample of a normal average test subject. This hinders accurate diagnosis. Such a problem can be avoided by configuring each flow channel in a multilevel structure as described above. Specifically, it effectively eliminates a problem in the introduction of a blood sample into a micro flow channel, and reproduces a smooth flow in the micro flow channel for a blood sample of a healthy test subject and reproduces the occluded state which corresponds to the factor for a blood sample of a test subject exhibiting a certain sign of disease, for example, thereby enabling accurate diagnosis and appropriate guidance for healthy lifestyle habits. For instance, the depth of the flow channel in the first substrate 10 may be a three-level structure of 300 µm, 100 µm and 30 µm.

By performing microfabrication in such a way that both of the first substrate 10 and the second substrate 20 have a multilevel structure, it is possible to realize a microchannel that reproduces capillary blood vessels, which is a model of microcirculation reproducing a more complex biological model. Providing shaping on both surfaces of substrates enables a complex structure which has been unrealizable because of processing technology and cost constraints, thus allowing more accurate diagnosis.

Although the cross-sectional shape of a micro flow channel is substantially rectangular in the microchannel array 100 described above, it is not limited thereto but can be different as appropriate. For example, the side wall of a groove can be tapered in the depth direction. If the side wall of a groove is tapered in the depth direction, the introduction of a blood sample into a micro flow channel is smoother, so that the adhesion of blood platelets on a micro flow channel is not recognized for a normal test subject, and the adhesion on a micro flow channel and the occlusion are recognized for a test subject with a certain sign of disease, thereby allowing accurate diagnosis. This also clarifies a difference among specimens in the measurement of the speed, number, deformability and so on of blood cells that deform and pass through a micro flow channel.

Furthermore, although the flow channel width, the flow channel depth and the flow channel length of the micro flow channel 7 are respectively uniform in the microchannel array 100 described above, it is not limited thereto but can be different as appropriate. If there are a plurality of different flow channel widths, flow channel depths and/or flow channel lengths of the micro flow channel 7, it is possible to change the shear stress acting on a blood sample. This enables the obtainment of larger information in a blood test method that measures the fluctuations in the number of blood cells at the inlet and the outlet of a micro flow channel, the occluded state of microgrooves by each component of blood, and a time period required for blood to pass through a micro flow channel and then obtains the flow characteristics or the activity of the blood component based on the measurement results. The detail is described later.

In addition, although the fluid inlet 1 and the fluid outlet 2 of a blood sample or the like are formed on the second substrate 20 in the microchannel array 100 described above, it is not limited thereto, and they may be formed on the first substrate 10. Further, although there are one fluid inlet and one fluid outlet in the above example, it is not limited thereto, and there may be a plurality of fluid inlets and a plurality of fluid outlets. Furthermore, the microchannel array 100 may have a plurality of measurement portions (a fluid inlet, a fluid outlet and an internal space structure connecting them). Alternately, the fluid inlet 1 and the upstream flow channel may be connected directly, without forming the inlet-side space 3. This is the same for the outlet-side space 4. In such a case, the same number of fluid inlets as the number of upstream flow channels may be formed and a given amount may be injected using a fluid injection control device or the like.
The size, the thickness and so on of substrates are not necessarily the same between the first substrate 10 and the second substrate 20, but may be different as appropriate. The shape of depressions, the shape of grooves, dimensions and so on are also not limited to those describe the above example, and they may be varied according to the purpose.

Although the application to a blood test is described as an example, the present invention is not limited thereto and it may be applied to other uses (for example, the measurement for obtaining information regarding cells).

### [Microchannel array manufacturing method]

A method of manufacturing a microchannel array according to this embodiment is described hereinafter. The case of using a resin as a material of a microchannel array is described. When using a Si substrate, the grooves, depressions and so on of the first substrate and the second substrate may be produced according to the description of the above-mentioned Patent document 1. Different materials may be combined for the first substrate and the second substrate as a matter of course.

The microchannel array of this embodiment is manufactured by fabricating a first substrate and a second substrate in a step of forming resist patterns on the substrates, a step of forming a metal structure through deposition of a metal over the resist patterns formed on the substrates, and a step of forming a molded article using the metal structure as a mold, and then adhering or joining the first substrate and the second substrate together.

A method of manufacturing a substrate for a microchannel which has a two-level structure (upstream flow channel or the like) in the depth direction is described hereinafter. In this method, a desired resist pattern is formed by:
(i) formation of a first resist layer on a substrate;
(ii) alignment of the substrate and a mask A;
(iii) exposure of the first resist layer with the use of the mask A;
(iv) heat treatment on the first resist layer;
(v) formation of a second resist layer on the first resist layer;
(vi) alignment of the substrate and a mask B;
(vii) exposure of the second resist layer with the use of the mask B;
(viii) heat treatment on the second resist layer; and
(ix) development of the resist layers.
   Then, a metal structure is deposited on the substrate by plating according to the resist pattern formed in the above process. After that, a resin molded product is formed by using the metal structure as a mold, thereby producing a microchannel array.

The resist pattern formation step is described in further detail below. For example, when forming a microgroove with a depth of 10 µm and a flow channel with a depth of 50 µm on a substrate, a first resist layer (50 µm in thickness) and a second resist layer (10 µm in thickness) are deposited on one another, and then each layer is exposed or exposed and heat-treated.

In the development process, a pattern with a depth of 10 µm to serve as the second resist layer is obtained firstly, and then a pattern with a depth of 50 µm to serve as first resist layer is obtained. In order to prevent the pattern with a depth of 10 µm, which is the second resist layer, from being dissolved or distorted by a developer in the formation of the pattern with a depth of 50 µm, it is required to control the solubility of each layer in the developer. When forming the resist layer by spin coating, it is possible to develop alkali resistance by adjusting a baking (solvent drying) time of the second resist layer.

One technique for developing the alkali resistance of a photodegradable positive resist is to increase a baking time (solvent drying time) so as to harden the resist. The baking time of the resist is normally adjusted according to the thickness of a layer, the density of a solvent such as thinner, and the sensitivity. Increasing the baking time can develop the alkali resistance.

Overbaking of the first resist layer hardens the resist too much, making it difficult to dissolve a light-exposed part and form a pattern in the subsequent development step. It is thus preferred to adjust baking conditions by reducing the baking time or the like. Equipment used for the baking is not particularly limited as long as it can dry a solvent, and an oven, a hot plate, a hot-air dryer or the like may be used.

Because the development of the alkali resistance is limited compared with chemically amplified negative resist, the combined thickness of resist layers is preferably in the range of 5 to 200 µm and more preferably in the range of 10 to 100 µm.

Besides the optimization of a baking time, another method for developing the alkali resistance of the chemically amplified negative resist is the optimization of the crosslink density. Normally, the crosslink density of a negative resist can be adjusted by an exposure amount. In the case of a chemically amplified negative resist, it can be adjusted by an exposure amount and a heat treatment time. The alkali resistance can be developed by increasing the exposure amount or the heat treatment time. When using a chemically amplified negative resist, the combined thickness of resist layers is preferably in the range of 5 to 500 µm and more preferably in the range of 10 to 300 µm.

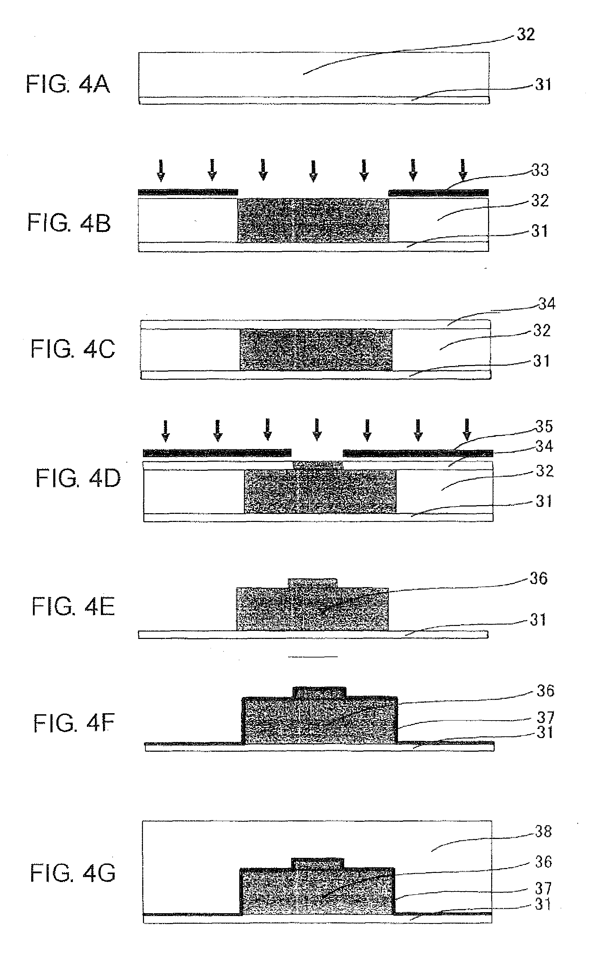

The steps (i) to (ix) are described in further detail hereinbelow.
(i) The formation of the first resist layer 32 on the substrate 31 is described below. Fig. 4A shows the state where the first resist layer 32 is formed on the substrate 31. The flatness of a resin microchannel array that is obtained by the molded product formation step is determined by the step of forming the first resist layer 32 on the substrate 31. Thus, the flatness when the first resist layer 32 is deposited on the substrate 31 is reflected in the flatness of a metal structure and the flatness of a resin microchannel array eventually. The flatness is significantly important for adhering or joining the first substrate 10 and the second substrate 20, and the use of optical conditions is preferred for ensuring the high flatness.

Although a technique to form the first resist layer 32 on the substrate 31 is not limited in any way, generally used techniques are spin coating, dip coating, roll coating, dry film resist lamination and so on. Particularly, the spin coating is a technique of depositing a resist on a spinning glass substrate and it has an advantage of very flat coating of a resist on a glass substrate with a diameter of more than 300 mm. The spin coating is thus preferred for use to achieve the high flatness.

There are two types of resists that may be used as the first resist layer 32: a positive resist and a negative resist. Because the depth of a resist that can be fomred changes depending on the resist sensitivity and exposure conditions, when using a UV exposure system, for example, it is preferred to select an exposure time and a UV output level according to the thickness and sensitivity of a resist. In the case of using a wet resist, there are a technique of changing the spin coating rotation speed and a technique of adjusting the viscosity in order to obtain a desired resist thickness with the use of spin coating, for example. The technique of changing the spin coating rotation speed obtains a desired resist thickness by controlling the rotation speed of a spin coater. The technique of adjusting the viscosity controls the resist viscosity according to the flatness level which is required for the actual use because the degradation of flatness can occur if a resist is too thick or a resist deposition area is too large. In the spin coating, for example, the thickness of a resist layer that is deposited at a time is preferably in the range of 10 to 50 µm and more preferably in the range of 20 to 50 µm so as to maintain the high flatness. Obtaining a desired resist layer thickness while retaining the high flatness can be achieved by forming a plurality of resist layers.

(ii) The alignment of the substrate 31 and the mask A 33 is described below. In order to set the positional relationship between the pattern of the first resist layer and the pattern of the second resist layer as designed, it is necessary to perform accurate alignment in the exposure using the mask A 33. The alignment may be made by a technique of providing cutting in the corresponding positions of the substrate 31 and the mask A 33 and fixing them with pins, a technique of reading the positions using a laser interferometry, a technique of creating position marks in the corresponding positions of the substrate 31 and the mask A 33 and performing alignment using an optical microscope, or the like. The technique of performing alignment using an optical microscope may create a position mark on the substrate by photolithography and create a position mark on the mask A 33 by laser beam equipment, for example. This technique is effective in that the accuracy within 5 µm can be easily obtained by manual operation using an optical microscope.

(iii) The exposure of the first resist layer 32 with the use of the mask A 33 is described below. Although the mask A 33 that is used in the step shown in Fig. 4B is not limited in any way, an emulsion mask, a chrome mask, or the like may be used. In the resist pattern formation step, the size and accuracy depend on the mask A 33 to be used. The size and accuracy are reflected in a resin molded product. Hence, in order to obtain a microchannel array with a given size and accuracy, it is necessary to specify the size and accuracy of the mask A 33. Although a technique of increasing the accuracy of the mask A 33 is not limited in any way, one technique is to replace a laser light source to be used for the pattern formation of the mask A 33 with the one having a shorter wavelength, for example. This technique, however, requires high facility costs, resulting in higher fabrication costs of the mask A 33. It is thus preferred to specify the mask accuracy according to the accuracy level which is required for the practical use of a microchannel array.

A material of the mask A 33 is preferably quartz glass in terms of the temperature expansion coefficient and the UV light transmission and absorption characteristics; however, because it is relatively expensive, the material is preferably selected according to the accuracy level which is required for the practical use of a resin molded product. In order to obtain a desired structure having different depths or heights as designed or a structure in which the first resist pattern and the second resist pattern are different, it is necessary to perform highly reliable design of mask patterns (transmitting/shielding parts) to be used for the exposure of the first resist layer 32 and the second resist layer 34. One approach for achieving this is to perform simulation with the use of CAE analysis software.

The light that is used for the exposure is preferably ultraviolet light or laser light for low facility costs. Although synchrotron radiation makes deep exposure, it requires high facility costs and thus substantially increases the price of a microchannel array, and therefore it is not industrially practical. Because the exposure conditions such as the exposure time and intensity vary by the material, thickness and so on of the first resist layer 32, they are preferably adjusted depending on a pattern to be formed. The adjustment of the exposure conditions is particularly important because it affects the accuracy and the pattern sizes such as the width and depth of a flow channel, and the interval, width (or diameter) and depth of a reservoir. Further, because the depth of focus varies by a resist type, it is preferred to select an exposure time and a UV output level depending on the thickness and sensitivity of a resist when using a UV exposure system, for example.

(iv) The heat treatment of the first resist layer 32 is described below. Annealing is known as the heat treatment to be performed after the exposure in order to correct the shape of a resist pattern. In this example, it aims at chemical crosslinking and is performed only when a chemically amplified negative resist is used. The chemically amplified negative resist is mainly composed of two- or three-component system. For example, a terminal epoxy group at the end of a chemical structure is ring-opened by exposure light and the crosslinking reaction is exerted by the heat treatment. If a layer thickness is 100 µm, for example, the crosslinking reaction progresses in several minutes by the heat treatment with a temperature of 100°C.

Excessive heat treatment on the first resist layer 32 makes it difficult to dissolve a non-crosslinked part to form a pattern in the subsequent development step. Thus, if a resist thickness is not more than 100 µm, it is preferred to adjust processing as appropriate such as reducing a heat treatment time or performing the heat treatment only on the second resist layer 34, which is formed later.

(v) The formation of the second resist layer on the first resist layer is described below. Fig. 4C shows the state where the second resist layer 34 is formed. The second resist layer 34 may be formed by the same process as the formation of the first resist layer 32, which is described in the step (i). When forming a resist layer using a positive resist by the spin coating, the alkali resistance can be developed by increasing a baking time about 1.5 to 2.0 times longer than usual. It is thereby possible to prevent the dissolution or distortion of the resist pattern of the second resist layer 34 at the completion of the development of the first resist layer 32 and the second resist layer 34.

(vi) The alignment of the substrate 31 and the mask B 35 is described below. The alignment of the substrate 31 and the mask B 35 is performed in the same manner as the alignment of the substrate 31 and the mask A 33, which is described in the above step (ii).

(vii) The exposure of the second resist layer 34 with the use of the mask B 35 is described below. The exposure of the second resist layer 34 with the use of the mask B 35 is performed in the same manner as the exposure of the first resist layer 32 with the use of the mask A 33, which is described in the above step (iii). Fig. 4D shows the exposure of the second resist layer 34.

(viii) The heat treatment of the second resist layer 34 is described below. The heat treatment of the second resist layer 34 is basically the same as the heat treatment of the first resist layer 32, which is described in the above step (iv). The heat treatment of the second resist layer is performed in order to avoid the dissolution or distortion of the pattern of the second resist layer 34 when the pattern of the first resist layer 32 is formed in the subsequent development step. The heat treatment enhances the chemical crosslinking to increase the crosslink density, thereby developing the alkali resistance. A heat treatment time for developing the alkali resistance is preferably selected from the range of 1.1 to 2.0 times longer than usual, depending on a resist thickness.

(ix) The development of the resist layers 32 and 34 is described below. The development in the step shown in Fig. 4E preferably uses a prescribed developer suitable for the resist to be used. It is preferred to adjust the development conditions such as a development time, a development temperature, and a developer concentration depending on the resist thickness and the pattern shape. Appropriate condition setting is preferred because an overlong development time causes a pattern to be larger than a given size, for example.

As a method of increasing the flatness accuracy of the top surface of a molded product or the bottom of a micro pattern, there are a method of changing the type of a resist (negative or positive) that is used in the resist coating to apply the flatness of the glass surface, and a method of polishing the surface of a metal structure, for example.

In the case of forming a plurality of resist layers so as to obtain a desired pattern depth, it is feasible to perform the exposure and development of the plurality of resist layers at the same time, or to form and expose one resist layer and further form and expose another resist layer, and then perform the development of the two resist layers at the same time.

The metal structure formation step is described herein in further detail. The metal structure formation step deposits a metal over the resist pattern that is formed by the resist pattern formation step and forms the surface having depressions and projections of a metal structure in accordance with the resist pattern, thereby producing a metal structure.

As shown in Fig. 4F, this step first deposits a conductive film 37 over the resist pattern. Although a technique of forming the conductive film 37 is not particularly limited, it is preferred to use vapor deposition, sputtering, or the like. A conductive material that is used for the conductive film 37 may be gold, silver, platinum, copper, aluminum, or the like.

As shown in Fig. 4G, after forming the conductive film 37, a metal is deposited over the pattern by plating, thereby forming the metal structure 38. Although a plating method for depositing a metal is not particularly limited, electroplating or electroless plating may be used, for example. Although a metal that is used is also not particularly restricted, nickel, nickel and cobalt alloy, copper or gold may be used, for example. The use of nickel is preferred because it is durable and less costly. The metal structure 38 may be polished depending on its surface condition. In this case, in order to prevent contaminations from attaching to a product, it is preferred to perform ultrasonic cleaning after the polishing. Further, it is also feasible to perform surface treatment of the metal structure 38 using a mold release agent or the like so as to improve the surface condition. The tilt angle of the metal structure 38 along the depth direction is preferably 50° to 90° and more preferably 60° to 87° in order to obtain a resin molded product without affecting its shape and with high efficiency. The metal structure 38 that is deposited by plating is then released from the resist pattern.

It is possible to build a family of the metal structure 38 in order to reduce its manufacturing costs. The family building is a reproduction technique that performs electroplating on a produced metal structure. In the manufacture of a microchannel array of the present invention, manufacturing costs can be reduced by producing a master metal structure in the form of a projected pattern, if a product has a projected pattern, and manufacturing a metal structure in the form of a depressed pattern by the family building.

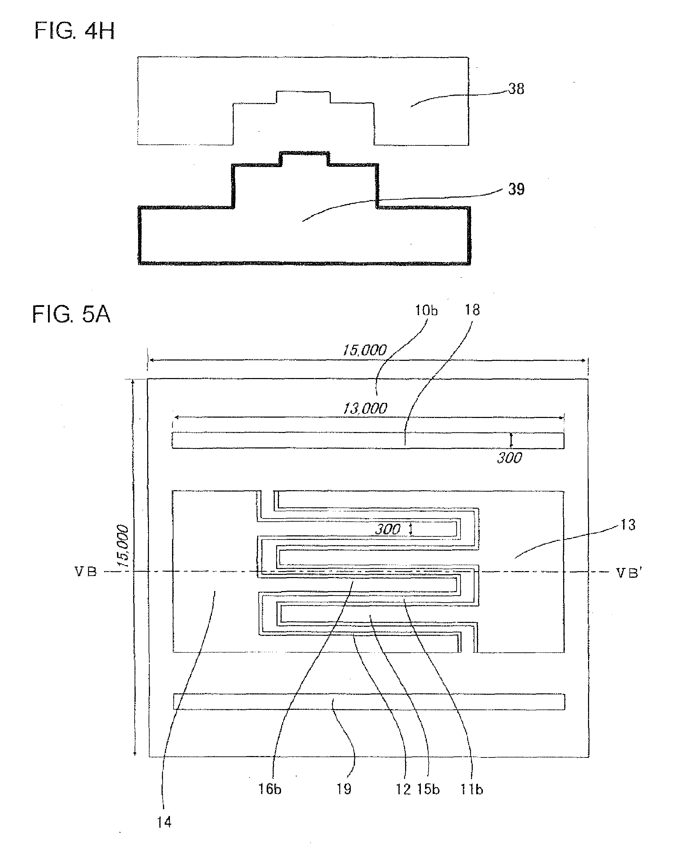

The molded product formation step is described hereinafter in further detail. The molded product formation step is a process of forming a resin molded product 39 with the use of the metal structure 38 as a mold as shown in Fig. 4H. Although a technique of forming the resin molded product 39 is not particularly limited, injection molding, press molding, monomer casting, solution casting, hot embossing, or roll transfer by extrusion molding may be used, for example. The use of the injection molding is preferred for its high productivity and pattern reproducibility. When forming a resin molded product by the injection molding using a metal structure with a given size as a mold, it is possible to reproduce the shape of a metal structure on a resin molded product at a high reproduction rate. The reproduction rate may be checked by using an optical microscope, a scanning electron microscope (SEM), a transmission electron microscope (TEM) and so on.

In the case of manufacturing a microchannel array with the use of a resin as a material of the first substrate 10 and the second substrate 20, it is possible to employ the injection molding which uses a master called a stamper. The injection molding using the stamper is a superior technique that is capable of achieving both high accuracy and low costs as used in the manufacturing of optical media.

In the structure that is described in the above-mentioned Patent document 1, it is necessary to form microgrooves and deep flow channels on a stamper to serve as a master and further provide a tilt angle which is required for mold release on each pattern depending on its depth. Providing complex shaping on a stamper causes not only an increase in manufacturing costs of the stamper but also the frequent occurrence of defects due to poor reproduction in the injection molding, resin fins upon mold release and so on, thus not suitable for practical use .

On the other hand, the manufacturing method of a microchannel according to this embodiment performs microfabrication on both of the first substrate 10 and the second substrate 20 and then adheres or joins the substrates together, thereby fabricating a microchannel array. It is thereby possible to produce a stamper having a simple pattern and perform injection molding for each of the first substrate 10 and the second substrate 20. This enables to reduce the manufacturing costs of a stamper and to perform the injection molding with a lowest possible defective rate such as poor reproduction and generation of resin fins upon mold release. It is thus a manufacturing method that is suitable for practical use.

A manufacturing method for creating a tilt in the shape in the flow channel depth direction of the upstream flow channel 5, the downstream flow channel 6 and/or the micro flow channel 7 of the first substrate 10 is described hereinafter. For example, in the case of employing the injection molding which uses a master called a stamper, the use of a photodegradable positive resist, for example, in the process of photolithography during manufacturing of a stamper enables creation of a tilt angle. If a photodegradable positive resist is used, the upper part of a projected pattern is exposed to a developer solution more deeply than the lower part, thus allowing easy creation of a tilt angle.

The semiconductor microfabrication technique that uses a silicon material has problems such as a high material cost of a silicon substrate, a high processing cost due to the necessity of performing photolithography for each substrate, and a varying dimensional accuracy of micro flow channels of each substrate. On the other hand, if the resin molded product 39 is formed by the injection molding with the use of the metal structure 38 having a given size as a mold, it is possible to reproduce the shape of the metal structure into the resin molded product 39 with a high reproduction rate. This is advantageous in being suitable for cost reduction (commercial production) by the use of a general resin material that allows material cost reduction and being capable of satisfying high dimensional accuracy.

The reproduction rate may be checked by using an optical microscope, a scanning electron microscope (SEM), a transmission electron microscope (TEM), a CCD camera and so on. By applying the quality control technique of optical discs, which has been actually put into commercial production, to a resin microchannel array, it is possible to manage and control various dimensional data, substrate flatness data, internal remaining stress data and so on based on standard deviation in lots of several tens of thousands.

In the case of producing the resin molded product 39 with the use of the metal structure 38 as a mold by the injection molding, for example, 10,000 to 50,000 pieces or even 200,000 pieces of resin molded products may be obtained with one metal structure 38. It is thus possible to largely eliminate the costs for producing the metal structures 38. Besides, one cycle of the injection molding takes only 5 to 30 seconds, being extremely efficient in terms of productivity. The productivity further increases with the use of a mold that is capable of simultaneous production of a plurality of resin molded products 39 in one injection molding cycle. In this molding process, the metal structure 38 may be used as a metal mold; alternatively, the metal structure 38 may be placed inside a prepared metal mold.

A minimum value of the flatness of the resin molded product 39 is preferably 1 µm or higher so as to enable easy industrial reproduction. A maximum value of the flatness of the resin molded product is preferably 200 µm or lower so as not to cause a problem when adhering or laminating the molded product 39 with another substrate. The dimensional accuracy of the pattern of the resin molded product is preferably within the range of ± 0.5 to 10% so as to enable easy industrial reproduction.

The dimensional accuracy of the thickness of the resin molded product 39 is preferably within the range of ± 0.5 to 10% so as to enable easy industrial reproduction. The thickness of the resin molded product 39 is not particularly specified, but it is preferably within the range of 0.2 to 10 mm in order to prevent breakage at removal during the injection molding, or breakage, deformation or distortion during handling. The size of the resin molded product 39 is also not particularly specified, and it is preferably selected according to usage, such as within the range of 400 mm in diameter when forming a resist pattern by lithography and if the resist layer is deposited by spin coating, for example.

In the case of using a plastic material as a material of a microchannel, the wettability of a plastic surface is modified according to need as described above. Techniques to modify the wettability of a plastic surface are classified broadly into chemical treatment techniques and physical treatment techniques. The chemical treatment techniques include chemical agent treatment, solvent treatment, coupling agent treatment, monomer coating, polymer coating, steam treatment, surface grafting, electrochemical treatment, anodic oxidation and so on. The physical treatment techniques include ultraviolet irradiation treatment, plasma contact treatment, plasma jet treatment, plasma polymerization treatment, ion beam treatment, mechanical treatment and so on.

Some of the modification techniques are characterized by developing adhesive properties in addition to hydrophilic properties of a thermoplastic resin surface. Because this is sometimes unfavorable for maintaining a large number of microgroove patterns on a microchannel array, it is necessary to select an appropriate modification technique depending on a required contact angle. Examples of applicable modification techniques are described below.

The chemical treatment techniques include inorganic and organic material coating. When using an organic material, a hydrophilic polymer in an aqueous solution, such as polyvinyl alcohol, is coated by dipping, spin coating or the like, and then sufficiently dried before use. If the hydrophobic property of a microchannel array is high, a uniform coating film thickness may not be obtained to cause variation in modification effects, and it is thus required to select an appropriate coating material. An example of a material that can be coated onto a hydrophobic surface is Lipidure-PMB (a copolymer of MPC polymer having phospholipid polar group and butyl acrylate), which is available from NOF CORPORATION.

Although this technique provides the modification effects with a relatively simple process without the need for a large-scale system and thus allows cost reduction, the modification effects can be deteriorated by ultrasonic cleaning or the like. It is thus preferred to increase the resistance to cleaning by coating a hydrophilic polymer after coating a material having an affinity for a material surface, or to use it for disposable applications.

The chemical treatment techniques also include steaming, particularly, vapor deposition. The vapor deposition is one of inorganic thin film deposition techniques, which heats and vaporizes a substance to be formed into a thin film in vacuum (with a pressure of 10⁻² Pa or lower) and deposits the vapor on an appropriate substrate surface. It enables processing at a relatively low degree of vacuum without the need for a large-scale system, thus allowing cost reduction.

The physical treatment techniques include plasma treatment, particularly sputtering. The sputtering accelerates positive ions that are generated by low-pressure glow discharge in an electric field and makes it collide against a cathode so that the substance on the cathode comes out and is deposited on the anode. The sputtering can deposit various kinds of materials, and the deposition of an inorganic material such as SiO₂ and Si₃N₄ at 10 nm to 300 nm allows hydrophilization of a material surface. This is also effective for a plurality of uses by repetitive ultrasonic cleaning or the like in that it has sustainable effects and provides repeatable test results. Further, it has no effluent and is compatible with cytotoxicity which is required for bioengineering applications or the like. The sputtering allows the thickness of a deposition film to be uniform, for example, the deposition of a SiO₂ film with a thickness of 10 to 50 nm allows achieving both transparency and hydrophilization.

When depositing an inorganic film on a microchannel array, sufficient degassing is required before sputtering in order to avoid that the microchannel array discharges absorbed moisture during the sputtering to cause a decrease in adhesion with the inorganic film. As other techniques for improving the adhesion of the resin surface and the inorganic film, there are a technique of performing etching with argon gas or the like on the surface of a microchannel array, and a technique of depositing an inorganic material with high adhesion, such as chromium, and then depositing a desired inorganic film. The sputtering requires a heat-resistant temperature of about 50°C to 110°C, and therefore it is essential to select the conditions such as (1) selecting a material having a glass transition temperature of higher than above temperature, such as polycarbonate, and (2) shortening a sputtering processing time (reducing a film thickness).

The physical treatment techniques include plasma treatment, particularly implantation. In the implantation, molecules are activated by plasma, and radicals that are generated on a polymer surface recombine to form a new functional group on the polymer surface. The introduction of such a functional group creates the polymer surface having a new property.

The physical treatment techniques also include plasma treatment, particularly plasma polymerization treatment. This technique vaporizes an organic material that serves as a raw material of a polymeric material for vapor phase transition and then activates the organic material by electron collision excitation in plasma to cause polymerization reaction, thereby depositing a polymer coating on the substrate. The plasma polymerization method eliminates the need for a solvent, which can be an impurity, because it uses a vaporized material molecule, and allows easy control of a film thickness. Further, because there is no remaining monomer, it is compatible with cytotoxicity that is required for bioengineering applications or the like. The plasma polymerization treatment raises polymerization reaction by activating an organic material with electron collision excitation in plasma; on the other hand, the vapor deposition polymerization raises polymerization reaction by heat.

The physical treatment techniques also include ultraviolet treatment, particularly excimer UV treatment. In the hydrophilization of a thermoplastic resin, it requires a low heat-resistance temperature and it is thus applicable to polymethyl methacrylate with a glass transition temperature of 100°C.

The excimer UV treatment applies ultraviolet light with a center emission wavelength of 120 nm to 310 nm with the use of an excimer lamp that uses discharge gas such as argon, krypton and xenon. By the application of the high-energy ultraviolet light, the molecules on the resin surface dissociate and a light hydrogen atom is easily drawn to create a highly hydrophilic functional group such as OH, thereby increasing the wettability of the surface. This technique enhances not only the hydrophilic properties but also the adhesive properties as the amount of ultraviolet exposure becomes larger, which is sometimes unfavorable for maintaining a large number of microgroove patterns. It is therefore necessary to select an appropriate amount of exposure depending on a required contact angle.

Another technique for hydrophilization is to use a vinyl acetate resin (product name: "Exceval") that is available from KURARAY, CO., LTD, a polyvinyl butyral resin or the like as a molding material. In order to maintain a microgroove shape, it is necessary to use water-type fluid at a temperature of 70°C or lower and avoid long-time immersion in water.

The above technique may be applied not only to the resin microchannel array but also to a silicon plate that is fabricated using the semiconductor processing technology.

In the above manufacturing process, the first substrate 10 and the second substrate 20 having depressions, grooves and so on that form a desired internal space structure are fabricated. The first substrate 10 and the second substrate 20 are then adhered or joined in such a way that the surfaces with the depressions, grooves and so on face each other. A microchannel array is thereby manufactured.

Methods for making the alignment of substrates so as to set a desired positional relationship between the first substrate 10 and the second substrate 20 are as follows. As described above, there are a method of forming depressed or projected patterns on the surface of each substrate so that the substrates are adhered at high positional accuracy with these patterns fit with each other when placed on one another, a method of fixing the outer end portions of the substrates by jigs, a method of using positioning pins into through holes for fixation, a method of observing and adjusting the positions with the use of a CCD camera and a laser optical device, and so on. Particularly, the method of forming depressed or positional patterns on the surface of each substrate and then laminating the substrates together can shorten a time required for the alignment and is thus suitable for commercial production. The method of forming depressed or projected patterns on the surface of each substrate may use a technique of forming a resist pattern by photolithography or a technique of performing shaping on a substrate for resist coating or a metal structure by machine cutting, electric-discharge machining, wet etching or the like. The depth or height of the depressed or projected patterns that are formed on the surface of each substrate are preferably selected from the range of 0.1 to 1 mm depending on the outer shape of a microchannel array or the like, so as to prevent the substrates once laminated together from being detached from each other due to the warpage of a resin molded product or vibration.

The above-described method of manufacturing a microchannel array enables material cost reduction because it uses a general resin material. Further, the above method is suitable for commercial production because it manufactures a microchannel array with the use of a metal structure. Furthermore, the above method satisfies a high dimensional accuracy because it performs microfabrication on each of the first substrate 10 and the second substrate 20 and then adheres or joins the substrates together.

### [Blood test method using a microchannel array]

A blood test method with the use of a microchannel array according to this embodiment is described hereinbelow.

The blood test method with the use of the microchannel array according to this embodiment brings a sample that at least contains a blood sample (physiological saline, reagent, in addition to blood sample) to flow into the upstream flow channel 5, which serves as a main blood vessel, individually or simultaneously from the inlet of the microchannel and further introduces the sample into the micro flow channel 7, which imitates a capillary blood vessel serving as a branch. The method then measures the fluctuations in the number of blood cells at the inlet and the outlet of a micro flow channel, the occluded state of the micro flow channel due to each component of blood, and a time period required for blood to pass through a micro flow channel and thereby obtains the flow characteristics or the activity of blood components. Blood components are classified broadly into blood cell components and blood plasma components.

The flow characteristics or the activity of blood components that pass through the micro flow channel in the microchannel array exhibit various morphologies in accordance with the properties of blood cell components and blood plasma components. Based on the difference, the health condition and the development of lifestyle-related diseases (diabetes, brain infarction, arteriosclerosis and so on) of a test subject can be predicted.

The blood test method using the microchannel array can measure the deformability of red blood cells and the occluded state of micro flow channels to thereby obtain the activity of red blood cells. A red blood cell, which is one of blood cell components, has the function of carrying oxygen. Normally, the red blood cells in a living body are nearly produced in every three months. The diameter of a capillary blood vessel in a living body is about 6 µm, and a red blood cell, which has a diameter of about 8 µm, passes through the capillary blood vessel by being deformed, thereby carrying oxygen to end tissue. If the activity of a red blood cell is high, it exhibits high flexibility. For example, if a red blood cell flows into a micro flow channel with a width and depth of 6 µm, it is observed that the red blood cell is deformed and passes therethrough. Thus, the activity of red blood cells can be obtained by letting them flow through a micro flow channel.

If the occlusion of a micro flow channel occurs due to red blood cells, the presence of high blood glucose level or the presence of a sign of diabetes, which cause a decrease in the deformability of red blood cells, is predicted. The decrease in the deformability of red blood cells leads to hardening of the outer membrane of a red blood cell component, which results in a failure to pass through a micro flow channel with a width and depth of 6 µm as being deformed, for example. Generally, diabetic patients have red blood cells with hard outer membrane. The retinopathy and the necrosis of tissue, which are complications of diabetes, occur due to the occlusion of terminal microcirculation (capillary blood vessels) by red blood cells.

In the blood test with the use of the microchannel array, if a blood component which causes the occlusion of a micro flow channel is determined to be red blood cells, a doctor can provide an explanation about the probability of the development of diabetes to a test subject visually by showing the image of the occluded micro flow channel of a microchannel in addition to showing biochemical measurement data, for example, which is very persuasive in providing guidance for healthy lifestyle habits.

The blood test method using a microchannel array can measure the adhesibility of blood platelets onto a substrate surface and the occlusion of micro flow channels to thereby obtain the activity of blood platelets. Blood platelets, which is one of blood cell components, have the function of coagulating blood. The particle diameter is about 3 µm. If the activity of blood platelets is high, it exhibits high adhesibility, so that when bringing blood into a micro flow channel with a width and depth of 5 µm, for example, blood platelets are adhered onto the micro flow channel or in the vicinity of its exit, and further other blood cell components and fat components are adhered thereon, which leads to the occlusion of the micro flow channel.

It is predicted from the occurrence of occlusion of the micro flow channel due to the adhesion of blood platelets that there is a factor of the activation of blood platelets in a living body. In such a case, there is the possibility of narrowing blood vessels, hypertension and so on, and it is possible to provide guidance for healthy lifestyle habits in addition to showing biochemical measurement data. If the microchannel array has a plurality of micro flow channels with different sizes, a difference occurs in the shear stress acting on blood samples which pass through respective micro flow channels, which enables the obtainment of detailed data about the activity of blood platelets from a difference in adhesibility of the blood platelets. It is known that blood platelets exhibits higher agglutinability and are thus aggregated upon receiving a high shear stress, and a difference in the shear stress acting on a whole blood sample serves as a difference in the agglutinability of blood platelets, which can cause a change in the occluded state of the micro flow channel or a passage time of a whole blood sample.

When circulating through a body, blood platelets receive a high shear stress if there is a narrowed part of a blood vessel. Because the blood platelet aggregation due to the shear stress causes the generation of thrombus, it is very important to measure the sensitivity of the blood platelet agglutinability to the shear stress. Further, because the sensitivity of blood platelets to the shear stress varies by the intensity of the shear stress received in a body, it is effective for estimating the degree of narrowing blood vessels in the body. If a blood vessel in the body is largely narrowed, the sensitivity of blood platelets to the shear stress is high. If, on the contrary, a blood vessel in the body is not narrowed, the sensitivity of blood platelets to the shear stress is low. Generally, if the diameter of a capillary blood vessel is 6 µm and a flow rate therein is 1 mm/sec, the shear stress of a vessel wall is 4.66 x 10 dyn/cm². The aggregation of blood platelets begins to occur when the shear stress of about ten times larger than this value acts. Accordingly, with the use of a plurality of different flow channel widths and/or flow channel lengths of micro flow channels (for example, the widths and/or flow channel lengths of micro flow channels are set to 30 µm, 15 µm and 5 µm), it is possible to obtain detailed data of the sensitivity to the shear stress for each test subject. This allows providing guidance about lifestyle-related disease or the like based on the accurate diagnosis on the activity of blood platelets. When bringing the blood of a test subject into a micro flow channel and if the blood platelets are aggregated in the flow channel or in the vicinity of the exit of the flow channel due to the shear stress passing through the flow channel, it is assumed that there is a risk of developing diseases such as arteriosclerosis and cardiac infarction. There is thus the expectation for scientific elucidation by the accumulation of cases in this measurement.

The blood test method using the microchannel array can measure the adhesibility, deformability and size of white blood cells and the occluded state of a micro flow channel to thereby obtain the activity of white blood cells. The white blood cells have the function of repulsing foreign enemies such as virus coming from the outside by generating active oxygen. The particle diameter is about 12 to 14 µm. If the particle diameter of a white blood cell is as large as about 15 to 20 µm, the viral infection such as a common cold is predicted. If the flexibility for getting deformed to pass through the channel decreases, the activity decreases accordingly, which can lead to the degradation of the resistance to foreign enemies. Further, white blood cells exhibit higher adhesibility in a test subtract who has lifestyle habits such as stress, lack of sleep and smoking, and therefore the evaluation of the adhesion of white blood cells onto a material surface can be used as a guideline.

The blood test method using the microchannel array can measure the occluded state of a micro flow channel by a blood plasma component to thereby obtain the degree of presence of cholesterol in the blood plasma component. If the percentage of the presence of cholesterol in the blood plasma component is high, the viscosity of a blood sample increases, which causes a longer blood test time. Further, by confirming that a component causing the occlusion of the micro flow channel is cholesterol, it is possible to check the possibility of developing lifestyle-related diseases such as arteriosclerosis.

The blood test method using the microchannel array can measure the fluctuations of the number of each blood components at the inlet and the outlet of a micro flow channel, the occluded state of the flow channel due to each blood components, or a passage time of blood after fluorescently coloring blood cells or fluid components with a fluorescent material to thereby obtain the flow characteristics and the activity of each blood components of blood. The staining materials of blood cell components may be such that, white blood cells are stained with Rhodamine6G and blood platelets are stained with CFSE (Carboxyfluorescein Diacetate), for example, and then they are observed with a fluorescence microscope. This enables detailed measurement of the fluctuations of the number of each blood components and the occluded state of the flow channel due to each blood components, thus increasing the accuracy of diagnosis.

By using a high resolution camera that is capable of observing a wide range of 0.6 mm or larger vertically and horizontally and the image identification function in the above blood test method, it is possible to identify the passage, adhesion, occluded state and area of each blood components from a wide range of a microchannel array and obtain the characteristics for each blood sample. This enables more accurate observation. In the observation of the microchannel array with the use of a light microscope, the observation range when observing a blood sample which passes through micro flow channels is limited to several micro flow channels (for example, about 0.05 mm vertically and horizontally). Even if a specimen has high fluidity, blood aggregate can be generated upon contact with a material or air when collecting blood, and there is the possibility of erroneous diagnosis at the sight of the occlusion of the micro flow channel due to this.

With the development of image technology equipment, the observation range is as large as about 1 mm vertically and horizontally by the use of a high resolution CCD camera, for example, which allows the observation of a large range of, not a part of, a micro flow channel to determine its main morphology. By inputting the images of morphologies to serve as comparative references in advance to determine if the adhered material and area and the factor of the occlusion is caused by either of red blood cells, white blood cells, blood platelets, fat components and so on, thereby enabling determination to which morphology the result belongs. A method of displaying the test conclusions may display the fluidity of blood flowing through a micro flow channel, the adhesion, the blood component which causes occlusion, and an area by textual characters and further display the image of the morphology. If there are a plurality of identification morphology of the passage, the adhesion, the occluded state and the area of each blood components, a first morphology, a second morphology and a third morphology may be displayed together with its percentage. A preferred resolution of a CCD camera is about 3 µm in the observation range of 0.6 mm or larger vertically and horizontally, and it is preferred to use a camera with million or more pixels.

In the above-described blood test method, a period from the start of flowing of a blood sample to the completion of a certain amount of the sample may be digitally recorded, so that the passage, the adhesion, the occluded state and the area of each blood components are identified from images for each elapsed time, thereby obtaining the characteristics of each blood sample. A blood sample has various characteristics according to the lifestyle habits of a test subject. For example, the sensitivity of blood platelets to the shear stress varies, and the timing when the occlusion of a micro flow channel starts occurring due to the adhesion of cholesterol and blood cell components after the adhesion of blood platelets in the micro flow channel varies accordingly. By the digital recording of the period from the start of flowing of a blood sample to the completion of a certain amount of the sample and the image identification of the passage, the adhesion, the occluded state and the area of each blood components for each elapsed time, it is possible to keep track of the detailed characteristics of each specimen, thus increasing the accuracy of diagnosis.

In the above-described blood test method, it is possible to increase the accuracy when providing the guidance for prevention of lifestyle-related diseases to a test subject by displaying, printing and/or representing by voice the possibility of the development of a disease, the factor of lifestyle habits that affects the development of a disease, and the contents of the guidance for healthy lifestyle habits. Further, if a test subject keeps the image of the first measurement at home and then undergoes the blood test again after 6 months, for example, and another image is printed for comparison with the previous image, it is possible to visually recognize the effects of improving the lifestyle habits. This offers more practical understanding to raise the awareness for health.

The blood test method using the microchannel array can measure the migrability and the adhesibility of white blood cell fractions. Specifically, the method sets a difference in the concentration of a biologically active substance between the inlet and the outlet of a micro flow channel to enhance the movement of white blood cells through the micro flow channel, and then measures the fluctuations of the number of white blood cell fractions at the inlet and the outlet of the micro flow channel or in the flow channel and the occluded state of the flow channel due to the white blood cells, thereby obtaining the migrability and the adhesibility of white blood cell fractions. The way of flowing a blood sample enables the measurement of the migration of particular blood cells only with the difference in the concentration of a biologically active substance. Specifically, by setting a difference in the concentration of a biologically active substance, rather than a difference in hydrostatic pressure, between the inlet and the outlet of a flow channel, only the blood cells that are capable of recognizing the difference in the concentration of a biologically active substance migrate into the flow channel. The measurement of the number of blood cells and a passage time enables the blood test.

The blood test method using the microchannel array can measure the activity of blood cell components by coloring either of each blood cell components or fluid components with a fluorescent or luminescent substance and measuring the light intensity. With the optical system that applies light to the inlet and the outlet of the micro flow channel or the micro flow channel and the measurement of the variation of light reflected by or transmitted through the micro flow channel, it is possible to obtain quantitative data. The optical system to be used may be a fluorescence microscope, a laser microscope, a laser scanner or the like. By coloring either of each blood cell components or fluid components with a luminescent substance or identifying the light intensity emitted from each blood cell components, the identification between different kinds of blood cells and between blood cells and surrounding fluid is extremely facilitated. The use of a system program with a computer is preferred for an increase in measurement points and summarization and evaluation of measurement data.

The blood test method using the microchannel array can measure the activity of white blood cells by measuring the amount of chemiluminescence of white blood cells. The white blood cells have the function of repulsing foreign enemies such as virus coming from the outside by generating active oxygen. In a living body, the active oxygen and the antioxidation substance (SOD: Super Oxide Dismutase) are well balanced. If luminol chemiluminescence reagent is added to the whole blood, a difference between the active oxygen and the antioxidation substance in the blood can be obtained as the amount of chemiluminescence. Based on the combination of this measurement result with the measurement data of the antioxidation substance or the like, it is possible to keep track of the state of a living body such as viral infection and a balance with respect to the antioxidation substance.

The blood test method using the microchannel array can measure the activity of blood cell components by depositing a thin film such as gold on at least a part of the wall surface constituting the internal space structure of the microchannel array and measuring a change in the dielectric constant before and after passing through the micro flow channel as a change in the intensity of reflected light due to the surface plasmon resonance. A detection method using the surface plasmon resonance applies light to a thin-film plate such as gold by vapor deposition or the like and detecting a change in the dielectric constant on the surface of the thin film as a change in the intensity of reflected light at high sensitivity. The use of surface plasmon resonance equipment, which utilizes this phenomenon, for the measurement of reaction and coupling amount of biomolecules, which requires a very high sensitivity, and the kinetic analysis has been started. This method deposits a thin film such as gold on at least a part of the wall surface constituting the internal space structure of the microchannel array by vapor deposition or the like, detects the activity of blood cell components before and after passing through the micro flow channel as a change in the dielectric constant on the thin film surface (a change in the intensity of reflected light), and performs conversion to an electrical signal and amplification. In order to enhance a change in the dielectric constant on the thin film surface, a reagent may be fixed onto at least a part of the wall surface constituting the internal space structure of the microchannel array. The surface plasmon resonance sensor is micro-fabricated by the semiconductor processing technology, thus allowing the measurement by specifying the area of the micro flow channel.

The blood test method using the microchannel array can measure the activity of blood cell components by placing a sensor for detecting a small change in frequency by ultrasound on one of the wall surfaces which constitute the internal space structure of the microchannel array and measuring a frequency change before and after passing through the micro flow channel. The study for the application of the detection technique based on a change in frequency with the use of ultrasound to the detection of reaction or the like between biomolecules, which requires a very high sensitivity, has been made. The technique fixes a sensor for detecting a small change in frequency by ultrasound and an electrode on one of the wall surfaces which constitute the internal space structure of the microchannel array, detects the activity of blood cell components before and after passing through the micro flow channel as a small frequency change, and then performs conversion to an electrical signal and amplification. This allows a difference in the activity between specimens to take numerical form accurately. In order to increase the frequency change, a reagent may be fixed onto at least a part of the wall surface which constitutes the internal space structure of the microchannel array. An ultrasonic sensor is micro-fabricated by the semiconductor processing technology, thus allowing the measurement by specifying the area of the micro flow channel. Further, if the first substrate 10 which has the ultrasonic sensor is used repeatedly and the second substrate 20 is disposable, the test costs can be reduced.

The blood test method using the microchannel array can measure the activity of blood cell components by placing an ISFET sensor on one of the wall surfaces which constitute the internal space structure of the microchannel array and measuring a small electrical displacement before and after passing through the micro flow channel. The ISFET (Ion Sensitive FET) sensor covers the surface of a Si chip with a SiO² - Si³N⁴ film and amplifies the electrical change which occurs due to the chemical species adhered onto the surface with the use of a field effect transistor (FET). The study for the application of this technique to the detection of reaction or the like between biomolecules, which requires a very high sensitivity, has been made, and a supermicro glucose sensor or the like has been introduced.

The technique fixes the ISFET sensor and an electrode on one of the wall surfaces which constitute the internal space structure of the microchannel array, detects the electrical displacement before and after the passage through the micro flow channel, and then performs electrical amplification. In order to increase the amount of electrical displacement, a reagent may be fixed onto at least a part of the wall surface which constitutes the internal space structure of the microchannel array. Further, if the first substrate 10 which has the ISFET sensor is used repeatedly and the second substrate 20 is disposable, the test costs can be reduced.

The blood test method using a microchannel array can obtain biochemical data by placing an electrode and fixing a reagent on one of the wall surfaces which constitute the internal space structure of the microchannel array, mixing the blood with the reagent, and measuring the amount of a small electrical displacement after the chemical change. By observing the flow of blood components passing through the micro flow channel, the flow of the microcirculation (capillary blood vessels) in a living body is reproduced. The obtainment of the biochemical data is important when predicting the development of lifestyle-related diseases and providing the guidance for healthy lifestyle habits.

The biochemical data can be obtained in several hours in a facility for complete physical examination or the like because there is a sufficient measuring device. On the other hand, because a small practitioner's office does not have a measuring device, it takes several days when outsourcing the measurement. If the biochemical data such as cholesterol, liver function, uric acid and blood glucose level can be measured with the use of the microchannel array, it is possible to obtain a result speedily from a small amount of specimen. In a small medical practitioner class, this enables accurate prediction of the development of lifestyle-related diseases and provision of the guidance for healthy lifestyle habits.

The measurement of biochemical data is performed by fixing a reagent such as enzyme (e.g. glutamate oxidase) on at least a part of the wall surface which constitutes the internal space structure of the microchannel array, mixing it with a blood sample, then measuring the amount of a small electrical change after the chemical change through an electrode, and finally performing electrical amplification.

The blood test method using the microchannel array can obtain biochemical data by fixing a reagent onto at least a part of the wall surface which constitutes the internal space structure of the microchannel array, mixing the blood with the reagent, applying light thereto, and measuring the amount of the variation. A light source is preferably an infrared ray laser because it is capable of specifying a measurement range and accurately detecting the variation. After mixing the blood with the reagent, biochemical data can be obtained based on the variation of light reflection, transmission, absorption and reflected position.

The blood test method using the microchannel array according to the present invention is effective for animals also, and its development is expected. Examples of targets are domestic animals such as cattle (beef cattle and dairy cow) and pig. In order to observe the effect of living environment on those domestic animals, the microchannel array of the present invention may be applied in the same manner as the blood test for human. Pet animals such as dog and cat are examples of the targets. Presently, the pet animals are recognized as a member of family, and it is important for the families who live together for a long time to keep track of the effect of living environment on the animal. The microchannel array of the present invention can be used just like the blood test for human.

Because the blood test method with the use of the microchannel array according to this embodiment brings a blood sample into the upstream flow channel 5, which serves as a main blood vessel, from the inlet, which is a headstream, and further introduces the sample into the micro flow channel 7, which imitates a capillary blood vessel serving as a branch, further development of various applications are expected. The blood test method using the microchannel array can be used for a test tool for determining the effects of products such as health food, health drink and vitamin supplements. Further, by the provision of flow rate control systems at either one or both of the vicinity of an inlet and the vicinity of an outlet on a measuring device, an operator who carries out the blood test can repeatedly reproduce an optimum flowing state easily.

The blood test method according to this embodiment uses the microchannel array which models capillary blood vessels in a living body, and therefore it is possible to estimate the flow in the microcirculation system in a living body by observing the flow characteristics and activity of blood components flowing through a micro flow channel. This enables the prediction of the development of lifestyle-related diseases and provision of the guidance for healthy lifestyle habits based on the flow and occluded state. By actually observing the state of blood flowing through the micro flow channel in addition to the biochemical measurement data such as a blood glucose level, liver function and cholesterol, which are shown in a normal blood test, a test subject can really recognize the need to improve the lifestyle habits and become more interested in preventive medicine.

### [Examples]

Although the present invention is described in further detail hereinafter using examples, the scope of the present invention is not limited to the below-described examples.

A microchannel array that is made of a resin substrate is described hereeeinbelow.
A method of producing a resin molded product is described herein in detail with reference to Figs. 4A to 4H. As shown in Fig. 4A, the first resist coating was performed on a substrate with the use of an organic material ("PMER N-CA3000PM" manufactured by TOKYO OHKA KOGYO CO., LTD.). Then, a first resist layer was deposited as shown in Fig. 4B, and alignment was performed in such a way that a mask A having a desired mask pattern was placed on a desired position of the substrate having the first resist layer.

After that, with the use of a UV exposure system ("PLA-501F" manufactured by CANON INC. with the wavelength of 365 nm), light was applied from the side of the mask A for the exposure of the first resist layer. After the exposure, the first resist layer was heat-treated by heating the substrate using a hot plate (100°C for 4 minutes). After that, as shown in Fig. 4C, the second resist coating was performed on the substrate having the first resist layer with the use of an organic material ("PMER N-CA3000PM" manufactured by TOKYO OHKA KOGYO CO., LTD.).

Then, a second resist layer was deposited as shown in Fig. 4D, and alignment was performed in such a way that a mask B having a desired mask pattern was placed on a desired position of the substrate. Then, the second resist layer was exposed to light from the side of the mask B using the above UV exposure system. After the exposure, the second resist layer was heat-treated by heating the substrate using a hot plate (100°C for 8 minutes). After that, as shown in Fig. 4E, the substrate having the first resist layer and the second resist layer were developed to thereby form a resist pattern on the substrate (a developer: "PMER developer P-7G" manufactured by TOKYO OHKA KOGYO CO., LTD.).

Then, as shown in Fig. 4F, a conductive film was deposited on the substrate surface having the resist pattern. Specifically, sputtering was performed to thereby deposit a conductive layer, which is made of silver, on the resist pattern. After that, as shown in Fig. 4G, the substrate on which the conductive film was deposited was immersed in a nickel plating solution for electroplating to produce a metal structure (hereinafter referred to as a "nickel structure") in gaps in the resist pattern.

Finally, as shown in Fig. 4H, a plastic material was filled in the nickel structure with the use of the nickel structure as a mold by injection molding. A plastic molded product was thereby produced. A material that is used for the plastic molded product was acryl (PARAPET GH-S) manufactured by KURARAY, CO. LTD.

### [Fabrication of a comparative microchannel array X]

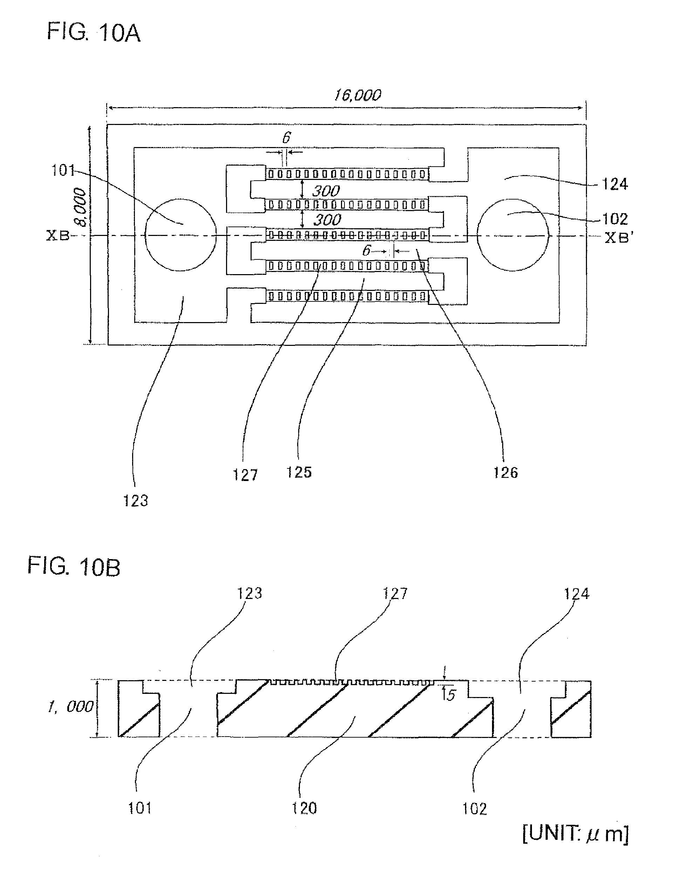

(Creation of a flow channel) Fig. 10A is a top view of a first substrate 120 of a comparative microchannel array X, and Fig. 10B is a cross-sectional view along line XB-XB' of the first substrate 120. As shown in Figs. 10A and 10B, the first substrate 120 has an inlet-side first depression 123, an outlet-side first depression 124, upstream first grooves 125, downstream first grooves 126.
As the first substrate 120, a silicon substrate (manufactured by Mitsubishi Materials Corporation) with a thickness of 1 mm and a diameter of 5 inches was used. It was produced as follows. First, aluminum, which serves as a mask, was deposited at 0.2 µm on the surface of the silicon substrate 120 by vapor deposition. Then, the patterning with aluminum was provided on the silicon substrate by photolithography. After that, the first dry etching (available from ULVAC, Inc.) was performed with the use of the aluminum pattern as a mask, thereby creating a flow channel with a width of 300 µm and a depth of 50 µm.

(Creation of a microgroove) The aluminum that was used for the first time was removed by a cleaning fluid. Then, the second aluminum deposition was performed on the surface of the silicon substrate 110. Further, the mask alignment was performed so that the upstream first grooves 125, the downstream first grooves 126 and microgrooves 127 were placed in desired positions with respect to each other. Then, the patterning with aluminum was provided on the silicon substrate by photolithography. After that, the second dry etching was performed with the use of the aluminum pattern as a mask, to thereby create a micro groove with a width of 6 µm and a depth of 5 µm. Then, the aluminum pattern was removed by a cleaning fluid, and through holes with a diameter of 1.6 mm are created by sand blasting at the left-end portion and the right-end portion, which serves as a fluid inlet 101 and a fluid outlet 102, respectively.

After that, the thermal oxidation was performed for the purpose of enhancing the hydrophilization so as to prevent adhesion of blood, and a SiO₂ film was formed on the silicon substrate surface. Then, a chip of 8 mm vertically and 16 mm horizontally was diced out by a dicing cutter and a transparent flat plate was placed thereon, thereby crating a space structure that allows a sample to flow through a microgroove. A contact angel with water was measured in the air. The measurement with the use of a contact angle measuring device ("CA-DT A model" manufactured by Kyowa Interface Science CO.,LTD.) resulted in 38°.
A transparent flat plate having the same size was placed on top of the first substrate 120. The microchannel array X was thereby fabricated.

### [Fabrication of a microchannel array A]

As a first substrate and a second substrate of a microchannel array A, the first substrate shown in Figs. 2A and 2B and the second substrate shown in Figs. 3A and 3B were used. The substrate was a resin substrate with a vertical dimension of 15 mm, a horizontal dimension of 15 mm, and a thickness of 1 mm.
(Production of the first substrate 10) The first substrate 10 as shown in Figs. 2A and 2B was produced by the method of forming a molded product shown in Figs. 4A to 4H. Specifically, the resist layer was formed by two times of resist coating and the exposure and heat treatment were performed thereon. After that, a conductive film was deposited on the substrate surface having the resist pattern as shown in Fig. 4F. Then, a metal structure was formed on the substrate on which the conductive film was deposited as shown in Fig. 4G. Then, a plastic molded product was produced by injection molding with the use of the metal structure as a mold as shown in Fig. 4H. In this process, six grooves, each having a width of 300 µm and a depth of 300 µm, were created on the substrate. Further, the first alignment portion 18 and the second alignment portion 19, each having a depressed pattern with a depth of 300 µm, were provided for the alignment.

(Production of the second substrate 20) The size of the substrate was the same as that of the first substrate 10. In the same manufacturing process as the first substrate 10, micro grooves with a width of 6 µm and a depth of 5 µm and so on were created as shown in Figs. 3A and 3B. Further, the third alignment portion 28 and the fourth alignment portion 29 were provided for the alignment. The height was 250 µm. The projected pattern was produced by previously creating a depressed pattern using wet etching on a glass substrate for resist coating that is used in the resist pattern formation step.

(Anti-blood adhesion processing) The surface modification was performed by plasma treatment on the first substrate 10 and the second substrate 20. A SiO₂ film was deposited at 100 nm with the use of a sputtering device (SV, manufactured by ULVAC, Inc.). The measurement of a contact angle with water just like the comparative microchannel array X resulted in 25°. The first substrate 10 and the second substrate 20 were laminated so that the alignment portions fit each other, and thereby the microchannel array A was fabricated.

### [Fabrication of a microchannel array B]

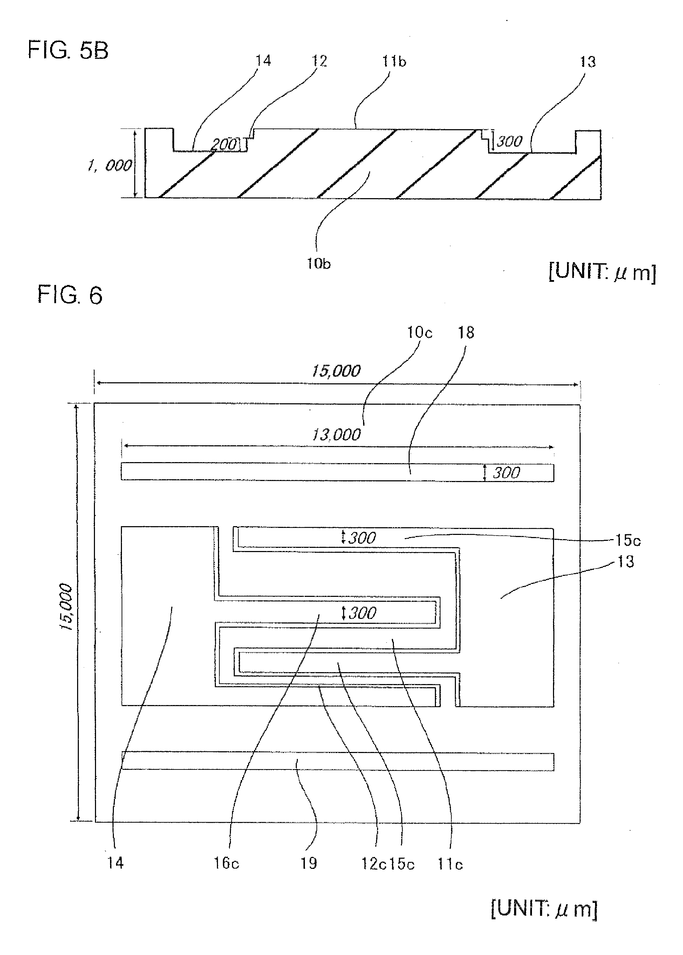

As a first substrate and a second substrate of a microchannel array B, the first substrate shown in Figs. 5A and 5B and the second substrate shown in Figs. 3A and 3B were used. In the following description, the same elements as in the above-described microchannel array A are denoted by the same reference numerals and the explanation was omitted as appropriate.
A first substrate 10b was produced to have two-level steps by the same manufacturing process as described above. A flow channel width of the upstream first grooves was 300 µm. A flow channel depth of first-level grooves 15b was 300 µm, and a flow channel depth of second-level grooves 12b was 100 µm. The other parts were the same as those of the above-described microchannel array A. The second substrate 20 was the same as the one used in the microchannel array A.

(Anti-blood adhesion processing) The surface modification was performed by plasma treatment on the first substrate 10b and the second substrate 20. A SiO₂ film was deposited at 100 nm with the use of a sputtering device (SV, manufactured by ULVAC, Inc.). The measurement of a contact angle with water just like the comparative microchannel array X resulted in 24°. The first substrate 10b and the second substrate 20 were laminated so that the alignment portions fit each other, and thereby the microchannel array B was fabricated.

### [Fabrication of a microchannel array B]

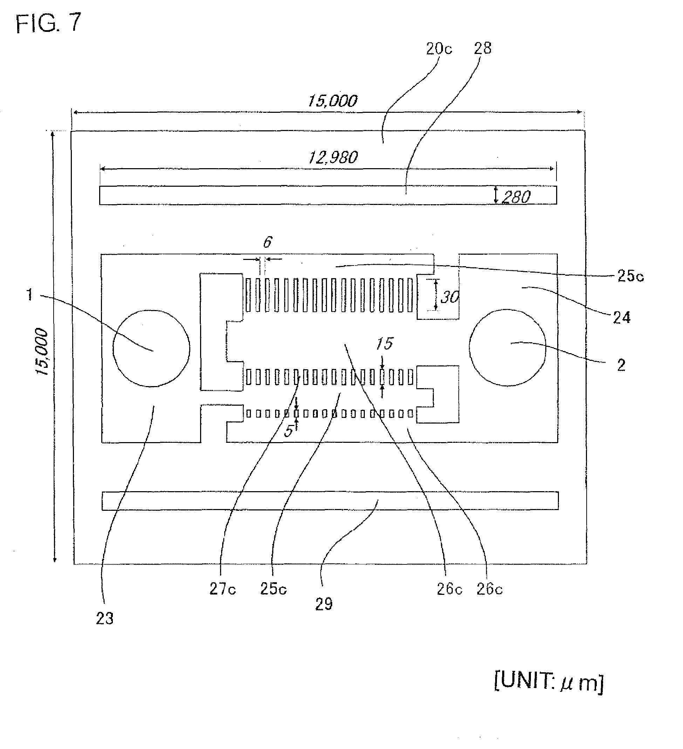

As a first substrate and a second substrate of a microchannel array C, the first substrate shown in Fig. 6 and the second substrate shown in Fig. 7 were used.

(Production of the first substrate) In the production of a first substrate 10c, a resist layer was formed according to the process shown in Figs. 4A to 4H, a flow channel width of the upstream first grooves was set to 300 µm. As shown in Fig. 6, a flow channel depth of first-level grooves 15c was 300 µm, and a flow channel depth of second-level grooves 12c was 100 µm. The other structure and manufacturing method were the same as those of the first substrate 10 in the above-described microchannel array A.

(Production of the second substrate) In the production of a second substrate 20c, a resist layer was formed according to the process shown in Figs. 4A to 4H, and a plurality of microgrooves 27 were created to include grooves with (a) a flow channel length of 30 µm and a depth of 5 µm, (b) a flow channel length of 15 µm and a depth of 5 µm, and (c) a flow channel length of 5 µm and a depth of 5 µm as shown in Fig. 7. The other structure and manufacturing method were the same as those of the second substrate 20 in the above-described microchannel array A.

(Anti-blood adhesion processing) The surface modification was performed by coating an organic material on the first substrate 10c and the second substrate 20c. The coating was performed with the use of a product: Lipidure-PMB (a copolymer of MPC polymer having phospholipid polar group and butyl acrylate) that is available from NOF CORPORATION. The measurement of a contact angle with water just like the comparative microchannel array X resulted in 18°. The first substrate 10c and the second substrate 20c were laminated so that the alignment portions fit each other, and thereby the microchannel array C was fabricated.

### [Fabrication of a microchannel array D]

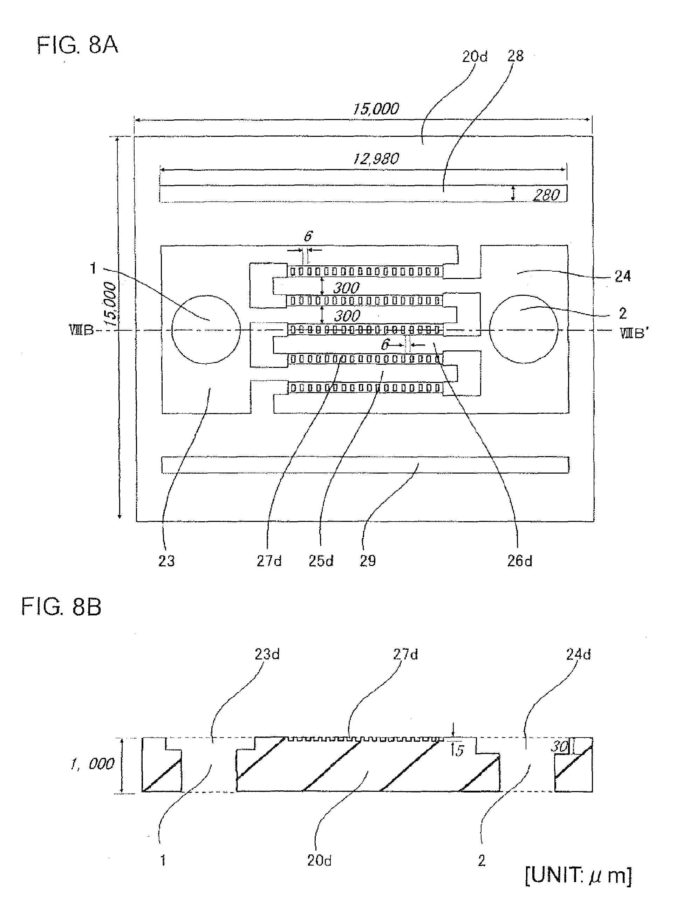

As a first substrate and a second substrate of a microchannel array D, the first substrate shown in Figs. 2A and 2B and the second substrate shown in Figs. 8A and 8B were used. The first substrate was the same as the first substrate 10 of the microchannel array A. The second substrate was manufactured by forming a resist layer according to the process shown in Figs. 4A to 4H and creating the microgrooves 27 with a groove width of 6 µm and having a two-level structure with groove depths of 5 µm and 30 µm. The other structure and manufacturing method were the same as those of the above-described microchannel array A.

(Anti-blood adhesion processing) The surface modification was performed by coating an organic material on the first substrate 10 and the second substrate 20d. The coating was performed with the use of a product: Lipidure-PMB (a copolymer of MPC polymer having phospholipid polar group and butyl acrylate) that is available from NOF CORPORATION. The measurement of a contact angle with water just like the comparative microchannel array X resulted in 16°. The first substrate 10 and the second substrate 20d were laminated so that the alignment portions fit each other, and thereby the microchannel array D was fabricated.

### [Comparative example 1]

Firstly, a blood test was performed with the use of the comparative microchannel array X. After immersing the comparative microchannel array X in physiological saline for the purpose of preventing the entry of air bubbles, it was set to a measuring module. Then, samples were introduced in the sequence of physiological saline and blood. The blood test performed the visual observation of a blood sample which was introduced from the inlet at the left-end portion and flowed through the flow channel and the microgroove and then exited from the right-end portion and a time required for the blood sample of 100 µl to pass therethrough.

The flow of the blood sample and the occluded state of the microgroove were observed with the use of a CCD camera.
The behavior of the blood sample was such that blood platelets started to adhere onto the surface before reaching the microgroove in 20 seconds from the start of passage, and the occlusion of the microgroove by aggregates of cholesterol and blood cells was observed in 30 seconds over a wide range. Therefore, it was unable to observe characteristics of the deformability and the adhesion of blood components such as red blood cells and white blood cells. A blood passage time was as long as 140 seconds, and it was probably caused by the adhesion of blood platelets onto the surface (material) before reaching the flow channel which was formed by the microgrooves 127. One of its causes was probably that a contact angle with water was as high as 38° in the hydrophilization processing for preventing blood adhesion. Another cause was probably that because the depth for introducing a blood sample into the flow channel formed by the microgrooves 127 was as small as 50 µm, the blood platelets were activated by the flow channel resistance and adhered onto the surface before reaching the microgroove.
Further, when using a silicon material, a method for preventing blood adhesion is typically the thermal oxidation that enhances the insulation resistance on the surface and reduces power consumption in the semiconductor fabrication; however, it is not optimum for a blood sample, particularly for anti-adhesion of each blood cells.

### [Example 1]

A blood test with the use of the above-described microchannel array A is described with the case of performing blood fluidity measurement. A blood sample is the same specimen as that used in the comparative microchannel array X.
Although the blood platelets started to adhere onto the wall surface before reaching the microgroove after 20 seconds from the passage of blood and the micro flow channel was occluded by the aggregates in the comparative microchannel array X, the adhesion of blood platelets did not occur under the same conditions in the microchannel array A. When using the microchannel array A, blood platelets adhered onto the wall surface after passing through the micro flow channel after 30 seconds from the passage of blood. It was observed that red blood cells and white blood cells, which are other blood cell components, were deformed and passed through the channel, thus exhibiting the morphology of normal blood components.
A passage time of blood was shortened to 60 seconds because of the absence of adhesion onto the surface before reaching the micro flow channel. In the comparative microchannel array X, blood adhered to the upstream flow channel 105 and the downstream flow channel 106 which imitated main blood vessels due to the adhesion onto a material surface and it failed to reproduce the microcirculation in a living body. On the other hand, the microchannel array A properly reproduced the microcirculation that models capillary blood vessels.
The reason for being able to suppress the adhesion of blood platelets onto a material is assumed that a stable SiO₂ film was deposited by sputtering and a contact angle with water was as low as 25°.

### [Example 2]

A blood test with the use of the above-described microchannel array B is described with the case of performing blood fluidity measurement. A blood sample is the same specimen as that used in the comparative microchannel array X.
Like the example 1, the adhesion of blood platelets onto the surface before reaching the micro flow channel did not occur, and the blood platelets adhered onto the surface after passing through the micro flow channel after 30 seconds. It was observed that red blood cells and white blood cells, which are other blood cell components, were deformed and passed through the channel, thus exhibiting the morphology of normal blood components.
A passage time of blood was 50 seconds, which is shorter than that in the example 1. Although the first substrate 10 of the microchannel array A that was used in the example 1 had the flow channel of one-level structure with a depth of 300 µm, the first substrate 10b of the example 2 had the flow channel of two-level structure with 100 µm and 300 µm, which allowed reproduction of the flow that imitated a living body with a main blood vessel (the flow channel with a depth of 300 µm), a branch blood vessel (the flow channel with a depth of 100 µm), and a capillary blood vessel (micro flow channel), thereby preventing the activation of blood platelets in the flow channel and enabling the observation after passing through the micro flow channel.

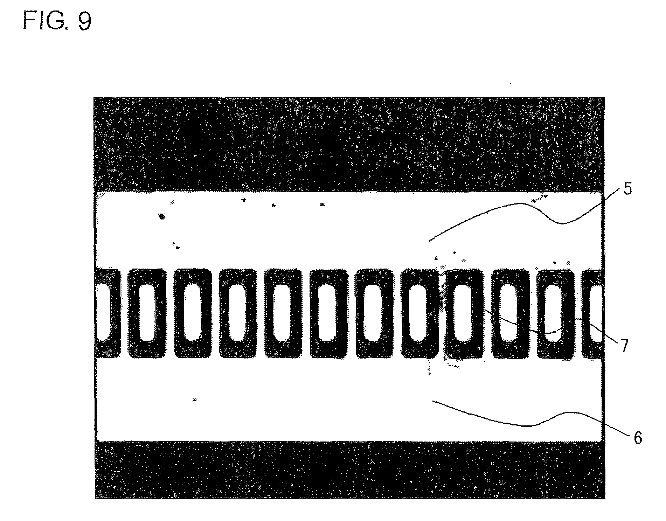

This blood test used a CCD camera (manufactured by CANON INC.) having an observation range of 1.2 mm vertically and horizontally and a resolution of two million pixels. The flow of a blood sample, the adhesion or occlusion due to each blood component, and so on were pre-input to a computer, and, after the blood test, the morphology of a typical flow state in the observation range of 1.2 mm vertically and horizontally was specified and the image was displayed properly. By utilizing a data processing speed and a memory capacity of the computer, it is possible to identify images for each blood passage time and display, print or represent by voice the disease prediction, the causes, and the items of lifestyle habits that should be improved based on the morphology. Fig. 9 is an image showing a blood flow in this measurement.

### [Example 3]

A blood test with the use of the above-described microchannel array C is described with the case of performing blood fluidity measurement. A blood sample was the same specimen as that used in the comparative microchannel array X.

The microchannel array C includes the flow channels that are formed by the microgrooves 27c and have three types of dimensions (a) a flow channel length of 30 µm and a depth of 5 µm, (b) a flow channel length of 15 µm and a depth of 5 µm, and (c) a flow channel length of 5 µm and a depth of 5 µm for the purpose of obtaining detailed information of the sensitivity of blood platelets of the blood of a test subject to the shear stress as described above.
The surface modification for anti-blood adhesion was performed by coating an organic material (the product name: Lipidure-PMB, a copolymer of MPC polymer having phospholipid polar group and butyl acrylate). Like the example 1, the adhesion of blood platelets onto the surface before reaching the micro flow channel did not occur in any of the flow channels of the microgrooves 27c. In the microgroove with a flow channel length of 5 µm and a depth of 5 µm, the adhesion of blood platelets onto the surface after passing through the flow channel formed by the microgrooves 27c was not observed until the end of passage. The start of the adhesion of blood platelets onto the surface after passing through the flow channel formed by the microgrooves 27d was observed after 30 seconds in the microgroove with a flow channel length of 30 µm and a depth of 5 µm and it was observed after 40 seconds in the microgroove with a flow channel length of 15 µm and a depth of 5 µm. A passage time of blood was 45 seconds.

Next, a blood test with the use of a specimen from another test subject was performed. The adhesion of blood platelets was started to be recognized after 15 seconds from the start of blood passage in the microgroove with a flow channel length of 30 µm and a depth of 5 µm, the adhesion of blood platelets was started to be recognized after 30 seconds in the microgroove with a flow channel length of 15 µm and a depth of 5 µm, and it was started to be recognized after 60 seconds in the microgroove with a flow channel length of 5 µm and a depth of 5 µm. Upon completion of blood passage, the microgroove with a flow channel length of 30 µm and a depth of 5 µm was almost occluded. A passage time of blood was 120 seconds.
It was confirmed from the above measurement results that if there are a plurality of flow channels formed by the microgrooves 27c having different shapes with different flow channel widths and depths, it is possible to observe the sensitivity of blood platelets of each specimen to the shear stress more accurately.

### [Example 4]

A blood test with the use of the above-described microchannel array D is described with the case of performing blood fluidity measurement. A blood sample was the same specimen as that used in the comparative microchannel array X. Like in the example 1, the adhesion of blood platelets onto the surface before reaching the flow channel formed by the microgrooves 27d did not occur, and the blood platelets started to adhere onto the surface after passing through the flow channel formed by the microgrooves 27d after 30 seconds. It was observed that red blood cells and white blood cells, which are other blood cell components, were deformed and passed through the channel, thus exhibiting the morphology of normal blood components.
A blood passage time was 55 seconds, which is slightly shorter than that in the example 1. Because the second substrate 20d of the microchannel array D that was used in the example 1 had the step with a depth of 30 µm at the front of the flow channel formed by the microgrooves 27d, it was possible to reproduce the flow that imitated a living body with a main blood vessel (the flow channel with a depth of 300 µm), a branch blood vessel (the flow channel with a depth of 30 µm), and a capillary blood vessel (the flow channel formed by the microgrooves 27d), thereby preventing the activation of blood platelets in the flow channel and enabling the passage through the microgroove.

By forming the various patterns on the first substrate and the second substrate of the microchannel array and laminating or adhering the substrates together, it is possible to realize the space structure having an extremely complex shape, thus providing a microchannel array that reproduces the microcirculation system (capillary blood vessel) of a living body.

### Industrial Applicability

The present invention may be applied to a microchannel array that is used for the measurement and evaluation of functions of red blood cells, white blood cells and blood platelets, which are formed components in blood.

## Claims

1. A microchannel array formed by adhering or joining a first substrate and a second substrate, having a fluid inlet and a fluid outlet on a surface, and internally having an internal space structure providing a connection from the fluid inlet to the fluid outlet, wherein the internal space structure comprises:
at least one upstream flow channel connected with the fluid inlet;
at least one downstream flow channel connected with the fluid outlet and located opposite to the upstream flow channel with a gap therebetween; and
a micro flow channel connecting between the upstream flow channel and the downstream flow channel, a minimum distance from a center of a sectional surface of the flow channel to a side wall of the flow channel being smaller than that of the upstream flow channel and the downstream flow channel,
each of surfaces of the first substrate and the second substrate to be adhered or joined together has grooves for creating the upstream flow channel and the downstream flow channel, and
the surface of the second substrate to be adhered or joined with the first substrate has a groove for creating the micro flow channel.

2. The microchannel array according to claim 1, wherein
a width and a depth of the upstream flow channel and the downstream flow channel are 20 µm or larger and 1000 µm or smaller, and a width and a depth of the micro flow channel are 1 µm or larger and 50 µm or smaller, and
a ratio of a width and a depth of a flow channel in each of the upstream flow channel, the downstream flow channel and the micro flow channel is within a range of 1:10 to 10:1.

3. The microchannel array according to claim 1 or 2, wherein
at least a part of the upstream flow channel, the downstream flow channel and the micro flow channel has a multilevel structure and/or a tilt structure in a depth direction.

4. The microchannel array according to claim 1, 2 or 3, wherein
the micro flow channel includes a plurality of micro flow channels with at least one of a width, a depth and a flow channel length being different.

5. The microchannel array according to any one of claims 1 to 4, wherein
the micro flow channel is located substantially orthogonal to the upstream flow channel.

6. The microchannel array according to any one of claims 1 to 5, wherein
a contact angle of a surface of the internal space structure with water is 0.5° or larger and 60° or smaller.

7. The microchannel array according to any one of claims 1 to 6, wherein
the first substrate and the second substrate are resin molded products.

8. The microchannel array according to any one of claims 1 to 7, wherein
the microchannel array is incinerable as industrial waste or infectious waste.

9. The microchannel array according to any one of claims 1 to 8, wherein
at least one of the first substrate and the second substrate is a transparent substrate.

10. A method of manufacturing a microchannel array comprising:
forming a first substrate and a second substrate by a step of forming a resist pattern on a substrate, a step of forming a metal structure by depositing a metal over the resist pattern formed on the substrate, and a step of forming a molded article using the metal structure as a mold; and
adhering or joining the first substrate and the second substrate.

11. The method of manufacturing a microchannel array according to claim 10, wherein
alignment is performed for providing a desired positional relationship when adhering or joining the first substrate and the second substrate.

12. A blood test method using the microchannel array according to any one of claims 1 to 9, comprising:
bringing a sample at least containing a blood sample to flow into a micro flow channel formed in an internal space structure in the microchannel array from a fluid inlet of the microchannel array;
measuring a state of each blood component of the blood passing through the micro flow channel; and
obtaining flow characteristics or activity of each blood component of the blood by the measurement.

13. The blood test method according to claim 12, wherein
the measurement of a state of each blood component of the blood is performed at least in close proximity to an inlet of the micro flow channel and in close proximity to an outlet of the micro flow channel.

14. The blood test method according to claim 12 or 13, wherein
(i) activity of a red blood cell as a blood component is obtained by measuring deformability when passing through the micro flow channel or/and an occluded state of the micro flow channel;
(ii) activity of a blood platelet as a blood component is obtained by measuring adhesibility on a side wall surface of the micro flow channel or/and an occluded state of the micro flow channel; or/and
(iii) activity of a white blood cell as a blood component is obtained by measuring adhesibility on a side wall surface of the micro flow channel, deformability when passing through the micro flow channel, a variation of a size, or/and an occluded state of the micro flow channel.

15. The blood test method according to claim 12 or 13, wherein
an occluded state of the micro flow channel due to a blood plasma component as a blood component is measured, and
a degree of presence of cholesterol in the blood plasma component is obtained.

16. The blood test method according to claim 12 or 13, wherein
the micro flow channel includes a plurality of micro flow channels having various shapes with at least one of a width, a depth and a flow channel length being different, and
adhesibility of a blood platelet as a blood component on the micro flow channel is measured for each of the plurality of micro flow channels having various shapes, and activity of the blood platelet is obtained from the measurement.

17. The blood test method according to one of claims 12 to 16, wherein
measurement is performed after fluorescently coloring either one of a blood cell and a fluid component of the blood with a fluorescent material.

18. The blood test method according to one of claims 12 to 17, wherein
at least one characteristics of the blood component is obtained by identifying passage, adhesion, an occluded state and an area of the blood component from a wide range of a microchannel array with use of a high resolution camera capable of observing a wide range of 0.6 mm or larger vertically and horizontally and an image identification function.

19. The blood test method according to one of claims 12 to 18, wherein
a period from a start of flow of the sample to an end of flow of a given amount of the sample is digitally recorded, and
at least one characteristics of a blood component of the blood is obtained by identifying passage, adhesion, an occluded state and an area of at least one blood component of the blood from an image for each elapsed time.

20. The blood test method according to claim 19, wherein
based on the obtained characteristics of a blood component of the blood, a possibility of development of a disease, a factor of lifestyle habits affecting development of a disease, or/and description of guidance for healthy lifestyle habits are displayed, printed or/and represented by voice.

21. A blood test method using the microchannel array according to any one of claims 1 to 9, comprising:
making a difference in concentration of a biologically active substance between an inlet and an outlet of a micro flow channel formed in the microchannel array to enhance movement of a white blood cell through the micro flow channel; measuring fluctuations in the number of white blood cell fractions at the inlet or the outlet of the micro flow channel or in the micro flow channel, or an occluded state of the micro flow channel due to a white blood cell; and obtaining migrability and adhesibility of a white blood cell fraction by the measurement.

22. A blood test method using the microchannel array according to any one of claims 1 to 9, comprising:
coloring either one of a blood cell and a fluid component of the blood with a luminescent or fluorescent substance;
bringing a sample at least containing a blood sample to flow into a micro flow channel formed in an internal space structure in the microchannel array from a fluid inlet of the microchannel array;
measuring light intensity of each blood component of the blood passing through the micro flow channel; and
obtaining activity of the measured blood component from a value of the light intensity.

23. The blood test method using the microchannel array according to claim 22, wherein
activity of a white blood cell as a blood component is obtained by coloring the white blood cell with a luminescent or fluorescent substance and measuring an amount of chemiluminescence of the white blood cell passing through the micro flow channel.

24. A blood test method using the microchannel array according to any one of claims 1 to 9, comprising:
depositing a thin film such as gold on at least a part of a wall surface of an internal space structure of the microchannel array, and bringing a sample at least containing a blood sample to flow into a micro flow channel formed in the internal space structure in the microchannel array from a fluid inlet of the microchannel array; and
measuring a change in dielectric constant before and after passing through the micro flow channel as a change in intensity of reflected light due to surface plasmon resonance, and obtaining activity of a blood cell component from a measurement value.

25. A blood test method using the microchannel array according to any one of claims 1 to 9, comprising:
placing a sensor for detecting a small frequency change by ultrasound on one of wall surfaces of an internal space structure of the microchannel array, and bringing a sample at least containing a blood sample to flow into a micro flow channel formed in the internal space structure in the microchannel array from a fluid inlet of the microchannel array; and
measuring a frequency change before and after passing through the micro flow channel, and obtaining activity of a blood cell component from a measurement value.

26. A blood test method using the microchannel array according to any one of claims 1 to 9, comprising:
placing a FET sensor on one of wall surfaces of an internal space structure of the microchannel array, and bringing a sample at least containing a blood sample to flow into a micro flow channel formed in the internal space structure in the microchannel array from a fluid inlet of the microchannel array; and
measuring a small electrical displacement before and after passing through the micro flow channel, and obtaining activity of a blood cell component from a measurement value,

27. A blood test method using the microchannel array according to any one of claims 1 to 9, comprising:
placing an electrode on one of wall surfaces of an internal space structure of the microchannel array and fixing a reagent; and
bringing a sample at least containing a blood sample to flow into a micro flow channel from a fluid inlet of the microchannel array to mix the blood sample with the reagent, measuring a small electrical displacement after a chemical change, and obtaining biochemical data.

28. A blood test method using the microchannel array according to any one of claims 1 to 9, comprising:
fixing a reagent onto at least a part of a wall surface of an internal space structure of the microchannel array;
bringing a sample at least containing a blood sample to flow into a micro flow channel from a fluid inlet of the microchannel array to mix the blood sample with the reagent, and applying light to the microchannel array; and
measuring a variation before and after light application and obtaining biochemical data.
